Europäisches Patentamt

European Patent Office

Office européen des brevets

(19)

(11)     EP 1 029 297 B1

(12)     EUROPEAN PATENT SPECIFICATION

(45) Date of publication and mention
of the grant of the patent:
**07.05.2003   Bulletin 2003/19**

(51) Int Cl.[7]: **G06F 17/50**

(21) Application number: **98955531.3**

(22) Date of filing: **04.11.1998**

(86) International application number:
**PCT/EP98/06968**

(87) International publication number:
**WO 99/023587 (14.05.1999 Gazette 1999/19)**

(54) **METHOD OF VIRTUAL RETRIEVAL OF ANALOGS OF LEAD COMPOUNDS**

VERFAHREN ZUM VIRTUELLEN WIEDERAUFFINDEN VON ANALOGA VON
FÜHRUNGSVERBINDUNGEN

PROCEDE D'EXTRACTION VIRTUELLE D'ANALOGUES DE COMPOSES DE TETES DE SERIE

(84) Designated Contracting States:
**AT BE CH CY DE DK ES FI FR GB GR IE IT LI LU
MC NL PT SE**

(30) Priority:  **04.11.1997  EP 97402620**

(43) Date of publication of application:
**23.08.2000   Bulletin 2000/34**

(73) Proprietor: **Cerep
75007 Paris (FR)**

(72) Inventor: **HORVATH, Dragos
F-59000 Lille (FR)**

(74) Representative: **Becker, Philippe et al
Cabinet Becker & Associés
35, rue des Mathurins
75008 Paris (FR)**

(56) References cited:
EP-A- 0 790 567          WO-A-91/10140
WO-A-97/27559          WO-A-98/47087
US-A- 5 463 564

• KING R D ET AL: "COMPARISON OF ARTIFICIAL
INTELLIGENCE METHODS FOR MODELING
PHARMACEUTICAL QSARS" APPLIED
ARTIFICIAL INTELLIGENCE, vol. 9, no. 2, March
1995, pages 213-233, XP002071862
• PICKETT ET AL: "diversity profiling and design
using 3d pharmacophores : pharmacore-derived
queries (PDQ)" JOURNAL OF CHEMICAL
INFORMATION AND COMPUTER SCIENCES.,
vol. 36, 1996, pages 1214-1223, XP002062096
WASHINGTON US
• MARTIN Y C: "3D DATABASE SEARCHING IN
DRUG DESIGN" JOURNAL OF MEDICINAL
CHEMISTRY, vol. 35, no. 12, 12 June 1992, pages
2145-2154, XP000676670
• GORDON E M ET AL: "APPLICATIONS OF
COMBINATORIAL TECHNOLOGIES TO DRUG
DISCOVERY 2. COMBINATORIAL ORGANIC
SYNTHESIS, LIBRARY SCREENING
STRATEGIES, AND FUTURE DIRECTIONS1"
JOURNAL OF MEDICINAL CHEMISTRY, vol. 37,
no. 10, 13 May 1994, pages 1385-1401,
XP000605162

**Description**

The invention relates to the combinatorial chemistry, and methods to synthesize and retrieve combinatorial products with the expected biological or physical properties, and more specifically to the molecular data management strategies, and more specifically to the molecular data management strategies applied in order to enhance the success rate of the discovery process of active compounds.

Computational approaches used to restrain the number of possible candidates to be subjected to activity tests, such as Virtual Screenings algorithms, which evaluate similarity scores between each compound of a database and the reference or lead compound and retrieve such molecules, are already known. In particular, prediction of structures of active analogs, starting from a learning set of compounds of known biological activities is a research field in full development, where many different approaches have been reported and tested, such as the simulation of the "docking" of a ligand in a receptor site (Ajay & Murcko, J.Med.Chem., 388, pp. 681, 1995) or the free energy perturbation approaches (Kollmann, Chemical Revue 1993, pp. 2395, 1995), or the screening 3D approaches (Tripos Technical Notes, Vol.1, Nr.2 - Molecular Diversity Manager, October 1995, as well as the program Cerius2 Drug Discovery Workbench from "MSI" Inc, Molecular Simulations Incorporated).

In another approach, R.D. KING et al.: "COMPARISON OF ARTIFICIAL INTELLIGENCE METHODS FOR MODELING PHARMACEUTICAL QSARS", APPLIED ARTIFICIAL INTELLIGENCE, vol. 9, no. 2, March 1995, pages 213-233 describes an attribute vector in which, for each group (e.g. $OCH_3$) constituting a molecule, nine integer numbers are provided, each integer number describing a PCA (physico-chemical attribute, e.g. polarity).

Furthermore, in the PDQ approach of S.D. PICKETT et al.: "Diversity Profiling and Design Using 3D Pharmacophores: Pharmacore-Derived Queries (PDQ)", JOURNAL OF CHEMICAL INFORMATION AND COMPUTER SCIENCES, vol. 36, 1996, pages 1214- 1223, a molecule descriptor exploits only a molecule's three central pharmacophores; the descriptor of a molecular compound thus specifies in a bit string both the 3D position and the type of these three pharmacophores, also taking into account of the conformational flexibility of the molecular compound since it is known that the pharmacologic properties of a molecule change as the molecule explores available conformational space. However, this 3D molecule descriptor attempts to describe a compound as a whole, and not in terms of building blocks.

However, the instant problem to be solved is the retrieval of potentially active analogs that have a similar pharmacophoric distribution to the one of molecules of biological activity out of the large collection of hundreds of millions of combinatorial products, synthesized on hand of building blocks of a reference library and of available chemical know-how, in order to select a biased sublibrary having a maximum content in active compounds and which can be synthesized and tested with respect to the presumed biological activity.

Prior art approaches are either too time-consuming or non-realistic in order to provide both fast and accurate retrieval of active analogs that are chemically feasible.

Recently published 2D approaches can be used to describe large libraries of molecules in term of connectivity descriptors, such as the issue by Higgs, Bemis, Watson & Wikel in J.Chem.Inf.Comp. Sci. Vol 37 n°5, pp. 861, 1997. However they do not account for geometrical and conformational aspects. Besides, approaches which analyze the molecular connectivity of large sets of candidates, are often flawed by their lack of realism in the description of the molecules.

More realistic approaches, like Tripos's approach, called « COMFA » (Comparative Molecular Fields Analysis), require an unambiguous superposition of the compared molecules, i.e. only fairly similar compounds (compounds having a common "template" or skeleton) can be meaningfully compared to each other. Furthermore, a great uncertainty remains on whether the calculated superposition mode of the compared molecules is physically relevant with respect to the binding mode to a receptor.

Furthermore, most of the prior art approaches perform retrieval of active analogs out of more or less random collections of products which oftenly leads to situations where the retrieved molecules are not chemically synthetisable, unstable or generally inadequate for use as drugs.

The present invention aims to solve these problems and propose a new approach based on an optimized trade-off between the degree of realism of the description of the molecules and the rapidity of retrieving them, by generating potential libraries that encode 3D multiconformational information under the form of pharmacophoric fingerprints of combinatorial products, and screening of these libraries by using scoring functions with a number of parameters specifically chosen.

More precisely, the object of the present invention is a method for rapidly retrieving similar, and therefore potentially active synthetic analogs of a lead compound, given a reference library of building block compounds and the synthesis protocols for the combinatorial synthesis of the analogs, wherein, at a first stage, the tens to hundreds of millions of chemically feasible combinatorial products are enumerated on the basis of available Building blocks and synthesis protocols; at a second stage the fingerprints of these compounds are generated on the basis of their 3D-multiconformational models ; at a third stage, the combinatorial products corresponding to the best-ranked fingerprints, are synthesized and the underlying assumption of the method - that these molecules will display physicochemical and biological properties that are similar to the properties of the reference molecules - is assessed by subjecting them to appropriate activity tests.

The method according to the invention comprises the steps of:

- selecting building blocks in the reference library by a chemical filter algorithm comprising elementary chemical rules, the selected building blocks being valid reaction partners in the chemical processes used in the combinatorial synthesis, detecting their reactive centers, and converting their molecular sketches into corresponding "radicals" (the substructures that make up the combinatorial products);
- building 3D-multiconformational models of the radicals by linking them temporarily to a bulky spacekeeper group and submitting the resulting complex to conformational sampling run yielding a collection of 3D conformers, then removing the spacekeeper in order to ensure that the reactive centers of the conformers are sterically accessible and its free valency points towards a region of free space previously occupied by the spacekeeper;
- registering these 3D-conformers, after verifying that they comply with certain sterical hindrance and conformational diversity criteria, into a 3D-radical database that serves as an input for a "ghost database" of combinatorial products, this "ghost database" comprising an algorithm emulating a database of combinatorial products in instantly generating the structure of any such product by linking together the registered conformers of the constituting radicals;
- for any molecular structure that is accessible within the ghost database, detecting any atom that displays physical property features of the pharmacophoric type on the basis of elementary rules accounting for the chemical nature of such an atom and the molecular environment in which it is placed, these pharmacophoric features being involved in determining the intensity of intermolecular interactions and comprising at least some of the hydrophobic, aromatic, hydrogen bond donor, hydrogen bond acceptor, anionic and cationic characters, and defining all the possible pairs of these pharmacophoric features such as (hydrophobic-hydrophobic), (hydrophobic-aromatic), (hydrophobic-hydrogen bond donor), etc., until (cation-anion), to be referred later on as "bipolar pharmacophores (BPs);
- for each bipolar pharmacore, calculating the distances between the pairs of atoms that represent the given BP, in every conformation of the considered molecule and creating a distance distribution density characterizing this BP;
- generating a conformational fingerprint vector that contains the distance distribution densities of all the bipolar pharmacores associated to a current conformation of the molecule, calculating an average molecular fingerprint from the conformational fingerprints of all the considered conformations and registering this average molecular fingerprint to constitute and supply a potential library,
- defining a scoring function to evaluate a measure of similarity between two molecular fingerprints, accounting for the relative importance of the pharmacophoric features owing to weighting factors that are calibrated in order to maximize a discriminative power with respect to different binding affinities; and
- generating the fingerprints of the lead compound, comparing these fingerprints to each molecular fingerprint of the potential library according to the above scoring function and selecting the molecules of the potential library for which the scoring function gives score values less than a specified threshold.

In particular embodiments, the weighting factors of the scoring function are calibrated in order to maximize the discriminative power between families of ligands of different receptors in a so-called « General Diversity paradigm », or to maximize the discriminative power between the compounds that bind to a given receptor, in contrast to those that have no binding affinity with respect to it, in a so-called « Receptor-Oriented Diversity paradigm ».

In a preferred embodiment, the method of the present invention includes, at the stage of generating the potential library, preliminary checking steps in order to discard the building blocks that can not be used as partners in any of the available synthesis protocols, either due to the absence of appropriate reactive groups or due to the presence of potentially interfering groups that may trigger unwanted side reactions, in order to prevent that the generated potential library contain compounds which could be formally represented as the coupling products of two BBs, but which for chemical reasons cannot be obtained in that way.

Furthermore, the chemical filter algorithms are not restricted to the recognition of reactive functional groups or interfering groups, but may include reactivity prediction models that use a set of original descriptors encoding the influences of electronic, steric and field effects on the reactive center, and which can be calibrated to optimally fit experimental reactivity data collected during the organic synthesis tests performed during the development of new synthetic protocols.

Therefore, a large majority of the molecules composing the "potential library" are actually synthetisable and represent pharmacologically acceptable species (without "exotic", very reactive or unstable groups), with the direct consequence that a majority of analogs retrieved by the virtual screening of the invention can be synthesized with little effort.

The method according to the present invention allows to design biased libraries that include the herein retrieved analogs, to synthesize these biased libraries and to evaluate the activities of their products. Furthermore, the herein generated structure-activity data can be used in order to improve the parametrization of the scoring function or to initiate new predictive approaches such as neural networks or decision trees, able to estimate the required activity of the molecule on the basis of its fingerprint.

Such data-mining approaches process previously collected structure-activity data, searching for relevant features that differentiate the active from the inactive compounds. In particular, hit-enriched sublibraries may be obtained by using a recursive partitioning method, consisting in establishing a classifying scheme representing the biological activity of a molecule, using the bipolar pharmacophore fingerprint vector,as molecular descriptor. Prior use of the recursive partitioning method in the discovery of active compounds mostly relied on the use of 2D and 3D molecular descriptors which do not encode the pharmacophoric properties of compounds and are therefore less relevant with respect to biological activity.

There are several advantages of present approach, in contrast to other drug design strategies and information management schemes in combinatorial chemistry.

The method according to the present invention can be integrated to a discovery paradigm that does no longer need a primary, "blind" screening of a compound library, if at least one ligand structure is known for the studied receptor.

Such paradigm includes the steps of generating and updating the potential library of fingerprints of combinatorial compounds, retrieval of potentially active analogs that are similar to known ligands, with respect to their pharmacophoric fingerprints out of this potential library, on the basis of the « General Diversity » paradigm, and design of a biased sublibrary, synthesis of the biased library and identification of active compounds, training and adjusting the parameters in order to define the « Receptor-Oriented Diversity » scoring function, or to use a data-mining approach in order to uncover the fingerprint element that are most relevant for the activity, retrieval and synthesis of other potentially active analogs according to the previously calibrated scoring function or to the method resulting from data-mining.

The screening of the conformer library can also be performed by recursive partitioning, when additional biological information is available on the lead compounds, as detailed below.

The potential library is directly linked to the building block stock databases, and updated in function of the changes in available building blocks and validated chemistries; this is a net advantage over the concept of virtual libraries which contain more or less random selections of compounds that may or may not be chemically feasible and/ or pharmacologically interesting.

The build-up of the fingerprints stored in the potential library takes profit of the extremely fast access to the multiconformational models of the combinatorial products from the combinatorial ghost database, which precludes the need of an explicit generation of three-dimensional for the up to 100 million product molecules; using one of the fastest conformational sampling programs, such the software "Catalyst" of MSI (Molecular Simulations Incorporated) that is claimed to process up to 10.000 compounds per 24 CPU hours, largely more than 1000 days would be required to complete such a task.

The combinatorial ghost database offers immediate access to the multiconformational models of any combinatorial product and instantly generates them by linking together the registered conformers of the radicals that constitute this product, and performing a 2 or 3-step torsional angle driving around the newly formed bond. The obtained conformers of the product are free of interatomic clashes, due to the precautions taken when modeling the radicals prior to their registration in the ghost database.

An explicit check of the quality of the geometries obtained by the coupling is nevertheless performed, in spite of those precautions. This operation is hardly more time-consuming than the input of molecular data files of the product : access to the structures of (MxN) combinatorial products of a combined type that can be obtained out of M BBs of a first type and N BBs of a second type is gained at the cost of a conformational sampling effort required to obtain the 3D-models of the (M+N) BBs.

The generated fingerprints represent distance distribution densities between pairs of atoms matching a pair of given pharmacophoric features. The rule-based identification of the atoms displaying given pharmacophoric features being very fast, the generation of the fingerprints of 100 million compounds would take some tens days depending on the processor operating system and its peripheral, for instance an estimated 20 to 30 days on a Unix workstation. They can be used to describe individual conformations, molecules, as well as collections of molecules within a unified formalism. Histograms corresponding to these fingerprints can be straightforwardly plotted and interpreted.

Another object of the invention is a "potential" library of potential combinatorial product fingerprints, wherein each potential combinational product is represented by its molecular fingerprint vector obtained as disclosed here above, and by identification codes of the two radicals that compose this product.

Other advantages and features of the present invention will be disclosed in the hereafter detailed description of non limitative embodiment in reference with the annexed figures which respectively show :

- Figure 1, a schematic flowchart of a first steps series of an exemplified method according to the invention and relative to a chemical algorithmic filter to select couples of radicals;

- Figure 2, a schematic flowchart of the following step series of such a method relative to a geometrical algorithmic filter to provide and register 3-D models of the radicals as input to a ghost data base;
- Figure 3, the screening of a focused library for binding to DAT;
- Figure 4, the general formula (I)
- Figure 5, the structure of compounds (A)-(D)
- Figure 6, the structure of compound S1;
- Figure 7, the structure of compounds (E)-(K)
- Figure 8, the similarity scores with respect to reference ligands: On Y - the log $K_i$ of the molecules, presumed to be a large positive value for the inactives, on X - the ranking of the compounds in function of their similarity score with respect to the reference. Ideally, the molecules with the lower log $K_i$ values should be ranked first.

The construction of potential libraries according to the invention relies on the collection of building blocks, referred as BB, currently available from a library and a set of synthesis protocols, both of which are regularly updated. Each such update automatically triggers the update of the potential libraries. A database containing the molecular 2D-sketches of BB, furnishes a description of the molecular connectivity of the BB.

Each synthesis protocol preferably requires, in the initial BB molecules, the presence of appropriate functional groups and the absence of potentially interfering reactive groups which may lead to side reactions. A preliminary algorithm selects its required molecules in respect of their chemical compatibility defined in each "reactivity profile" depending on the considered synthesis protocol, the chemical properties of the ligands of the receptor or the binding ability to the receptor. Such an algorithm is in the scope of the art of the skilled person.

Furthermore, each synthesis protocol may involve functional "transformers" to be appended to a first BB, prior to its coupling to a second BB. Thus, the chemical reactions hereafter considered are:

- either direct coupling processes between building blocks, such as for instance, with R1-COOH as a first BB, and $H_2$N-R2 as a second BB:

$$R1\text{-COOH} + H_2\text{N-R2} \rightarrow R1\text{-CO-HN-R2} + HOH$$

- or coupling with a functional transformation of the first BB prior to the coupling to the second BB, such as in:

$$R1\text{-NH}_2 + \text{Im-CO-Im} \rightarrow R1\text{-NH-CO-Im} + HIm \text{ (Im=imidazole)}$$

$$R1\text{-NH-CO-Im} + H_2\text{N-R2} \rightarrow R1\text{-NH-CO-NH-R2} + HIm$$

Transformers (the carbonyl group -C(=O)- in the above example) replace the original reactivity by a new one, opening the possibility to use the modified BBs in synthesis that are not feasible with the original ones.

A first algorithmic filter, referred to as the chemical filter, is implemented in order to check whether each BB qualifies for a given reaction, according to reactivity specifications listed in the corresponding synthesis protocol.

The chemical filter is used to select two subsets of BBs of type A and respectively B, which are considered to be valid reaction partners, to yield products of the type A-T-B, with T being the transformer, if any, required by the considered chemical assembly strategy. It involves preliminary steps to « clean » the BBs in removing accompanying counterions and cutting away the leaving groups to form radicals in which the reactive center is identified as such.

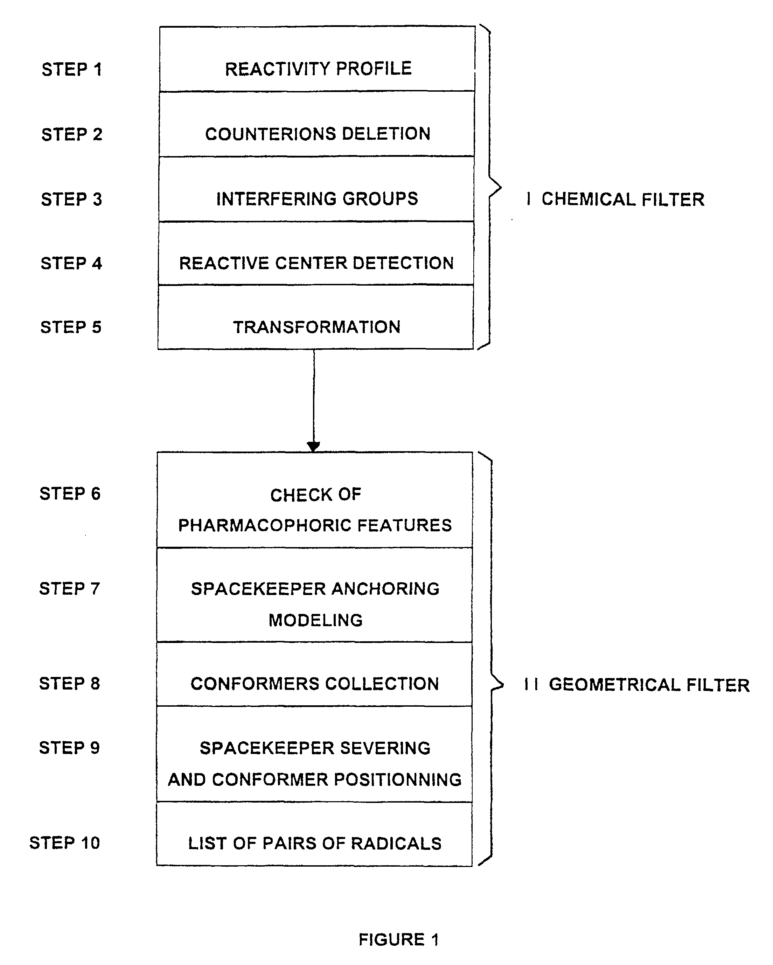

An example of a chemical filter 1 is illustrated in figure 1. It comprises the following steps :

Step 1 : scanning the so-called "reactivity profile" of the current synthesis, in order to input:

- the specified required groups that make the synthesis possible and the interfering groups that may get involved in unwanted concurrent processes, in terms of the type of the reactive center or the degree of substitution of nucleophilic centers (e.g. primary and secondary monoaromatic amines),
- the protective groups that are eliminated, the transformer groups that need to be added,
- threshold criteria regarding the number of rotatable bonds and the molecular mass of BBs

and then specifying and opening the BB files to be input to the chemical filter.

Step 2 : checking if the selected BB contains a single molecule and deleting the counterions defined as connex subgraphs of lower size than the one representing the main compound (such as $R\text{-NH}_3^+,/Cl^-$) if any;

Step 3 : checking the presence of any interfering groups in the selected BB that should trigger secondary processes, and discarding such current BB;

Step 4 : checking the presence of the required functional groups in the BB, and figuring out which atom, called hereafter the reactive center, is involved in forming a bond with the second BB. A part of the BB, called the "leaving group", is eliminated during the reaction to prepare the reactive center to be linked, the step detecting and deleting the corresponding fragment.

For example : in a carboxylic acid in an amidification process, the leaving group -OH is deleted and the reactive center is set at the carboxyl carbon: R-C(=O)-OH → R-C*(=O)-, * labels the reactive center.

If the BB contains several potentially reactive groups, then all the possible reactive centers are enumerated and a selection of the correct one is conducted with auxiliary software or routine rules implemented by the skilled person, or the compound is discarded; if no reactive group is found, the compound is also discarded.

Step 5 : if a functional transformation is specified due to the fact that another reactive center is considered to be more tunable to the synthesis, the transformer fragment is attached to the previously detected reactive center and a new reactive center is located at the atom of the transformer fragment that will form a bond with the BB, as, for example, with a reaction of the type:

$$R-HN\text{*}-+-C(=O)- \rightarrow R-HN-C\text{*}(=O)-$$

Thus, the chemical filter transforms the raw structures of the BBs into corresponding fragments as they appear in a final product, in detecting the reactive centers, deleting the leaving groups and coupling to a transformer moiety when required. These fragments are referred to as "radicals" which are liable to be reaction partners referred to as S1 and S2, one of the valencies of the reactive center is labeled as the free valency, to be used for coupling with the partner radicals in order to obtain the final products.

In the following rule-based algorithm II, a geometrical filter as illustrated in figure 1, the construction of 3D-geometries from conformational sampling of selected radicals, are carried out such as to ensure that, in resulting geometries, the reactive centers of the radicals are sterically accessible, e.g. the free valency points towards a region of free space, in order to ensure that any radical can be concatenated with partner radicals without any clashes between the linked moieties which form the final product.

In order to achieve this, a bulky "spacekeeper" group linked to the selected radicals is used in connection with the conformational sampling. In the present embodiment (figure 1), the following steps are carried out.

Step 6: hydrogens being added to the heavy atom skeleton of the sketches of each radical, 6 pharmacophoric features: aromaticity, hydrophobicity, hydrogen bond donor or acceptor property, positive and negative charge of every atom, are checked and listed, considering the specific chemical groups involved and more particularly that

- aliphatic amino groups are under cationic form, while aromatic amines are taken as neutral;
- a special flag is used to signal the presence of imidazole rings, which may appear under physiological conditions, at neutral pH, under both protonated or unprotonated form,
- carboxylate, sulphonate, phosphate groups and tetrazoles are considered to be anions.

Step 7 : the 2D-sketch of the radical is anchored with its free valency to the following bulky spacekeeper group, the tris(triiodosilyl) methyl-entity:

$$R-HN-C\text{*}(=O)-+-C(SiI_3)_3 \rightarrow R-HN-C(=O)-C(SiI_3)_3$$

Step 8: the resulting 2D-sketch of this compound is submitted to a conformational sampling run, performed for instance by the "Catalyst" MSI software, yielding a collection of possible conformers of this compound.

Step 9: for each of the conformers obtained at step 8, the spacekeeper moiety is now severed and deleted, restoring the free valency of the radical which now points towards the empty region previously occupied by the spacekeeper,

Advantageously, steric hindrance criteria are then performed in order to check whether the spacekeeper strategy has managed to insure an appropriate accessibility for the reactive center for every conformation. Conformational diversity criteria are then applied in order to check whether the obtained geometries differ enough from each other in order to justify their simultaneous storage, such as for instance:

- a comparison of the interatomic distances in the different conformations in order to make sure that the retained conformers are not redundant,
- an energy criterion discarding higher-energy conformers found to have an almost identical geometry with respect to lower-energy conformations.

At step 9, for each retained conformer, a coordinate transformation in a 3-Dimensional OXYZ reference system is performed, in order to place the reactive center in the origin of the reference system and to align the free valency along the Z-axis. The coordinates of the conformers are stored.

Step 10: a list of all the potential compounds that are obtained by first'coupling each radical S1 of a first set with every one of its partners S2 is then generated.

This list serves as input for the fingerprint calculation of the products, being submitted as a query to the ghost database routines which effectively manage the "combinatorial explosion" of the product structures, by linking every conformer of the radicals of a first type to every conformer of the radicals of a second type, performing a 2 or 3-step torsional angle driving around the linkage bond, and submitting the such obtained product conformers to a fingerprint calculation module that evaluates the distances within the pharmacophoric pairs of the so generated conformer, as described in more details in the following ;

- in step 11, looping over all pairs listed at step 10, current pairs of radicals (S1,S2) constituting a combinatorial product are retrieved from the 3D radical database in which they had been stored at step 9, together with all their available conformers ($S1_1$,$S1_2$,...) and respectively ($S2_1$,$S2_2$,$S3_3$,...) and their lists of the pharmacophoric features of the composing atoms, established at step 6. A new bond is created between the reactive centers of these radicals as defined at step 4, by modifying the connection tables. The pharmacophoric features of the atoms involved in the new bound are reevaluated, since their chemical type have changed due to this chemical transformation;
- in step 12, each conformer S1i of the first radical S1 is linked with each conformer S2j of the second radical S2, in mirroring the coordinates of the latter with respect to the XOY plane, and translating them along the Z axis in order to restore the correct length of the newly formed bond between S1i and S2j; a 2 or 3-step rotation around the new axis is performed.

Therefore, the generated number of conformers of the coupling product equal the number of conformers of the first radical times that of the second radical, times the number of rotations around the newly formed bond;

- in step 13, in order to construct the distance distribution density of pairs of pharmacophoric features, a complete set of interatomic distances is evaluated for the current conformation of each dimer; for a pair of pharmacophoric features, defining a Bipolar Pharmacophore (BP), a distance distribution density histogram of the current conformer of the current compound is constructed by classifying the atom pairs which match the current BP into distance classes or distance "bins" in function of the interatomic distance between the two atoms and eventually counting the number of atom pairs found in each of the defined distance classes. For example, 12 distance classes can be defined as follows : distance class 1 contains all the atom pairs that are less than 5 Angstrom away, class 2 the atom pairs that are between 5 and 6 Angstrom away,...,class i the atom pairs that are between i+3 and i+4 Angstrom away, ... class 12 the atom pairs that are more than 15 Angstrom away. The assignment of an atom pair to a distance class is done in a "fuzzy" manner, in order to avoid classification artifacts. For example, an atom pair with a distance of 5.5 Angstrom will be assigned a 100% appurtenance to class 2, while a distance of 5.0 Angstrom would cause this pair to be equally considered as a member of both classes 1 and 2, with an equal contribution of 50% to each one of them. This classification and counting of pairs in each distance class i is repeated for all the possible pairs of pharmacophoric features (fa,fb);
- in step 14, a generated conformational fingerprint vector describing the current geometry is represented, in the present embodiment, with a (6x7)/2xl2=252-element vector, where (6x7)/2=21 is the number of combinations that can be obtained with the 6 pharmacophoric features f1 to f6 introduced supra (aromatic-aromatic, aromatic-hydrophobic, .... anion-anion), and 12 being the number of interatomic distances classes.

Each fingerprint element FP (fa, fb, i), fa and fb being one of the group f1 to f6, is equal to the number of atoms pairs matching a given pair of pharmacophoric features (fa,fb) and which are separated by a distance that falls within distance class i, as defined in step 13. For example, the element of the fingerprint FP (cation, aromatic, 1 ) counts the number of atoms pairs in which one is a cation, the other is an aromatic atom and the distance between them falls within the range 4 to 5 Angstroms.

A molecular fingerprint vector, the Fuzzy Bipolar Pharmacophore Autocoreglogram (FBPA), is obtained by summation of the conformational fingerprints, followed by norming this sum with respect to the numbers of considered conformers of the product. This fingerprint vector is stored in association with the identification codes of the two radicals which compose that product. The collection of all the fingerprints of all the possible coupling products between all BBs qualifies the initial library synthesis processes and defines a potential library.

The totality of the molecular fingerprint vectors stored on disk as previously discussed form the Potential Fingerprint Library (PFL). Steps 1-14 are undertaken each time a new chemistry protocol is adopted, opening the access to new combinatorial products, the fingerprints of which are to be added to the PFL, or are repeated for each of the previously available chemistry protocols at regular time lapses, in order to update the configuration of the PFL in function of the changes in available Building Blocks in the starting materials stock.

A comparison algorithmic filter IV is then performed to compare the fingerprints of the reference compounds with each fingerprint of the potential library, by evaluating their global similarity score, which is defined as a weighted average of the partial similarity scores per feature pair, obtained beforehand by comparing the distance distributions corresponding to each pair of features.

Weighting factors are introduced to represent the relative importance of the different pharmacophoric features, and are the tunable parameters of the method. Indeed, the different physical, chemical or biological properties are more sensitive to the presence of specific bipolar pharmacophores than others : the similarity of two compounds with respect to given feature pairs is more important than the fact that the two compounds differ with respect to other feature pairs. To reflect the relative importance of the feature pairs, the weighting factors weigh the relative importance of the partial scores in the calculation of the overall similarity score.

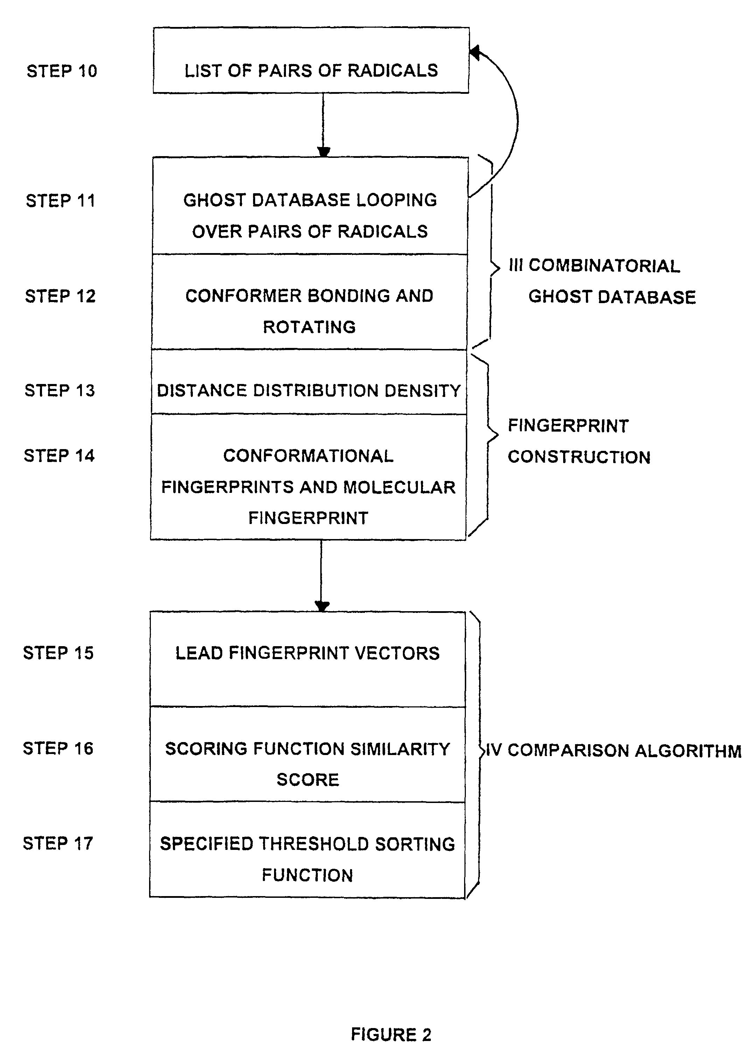

In the present embodiment, the comparison algorithm IV (figure 2) consists of:

- encoding, in step 15, of leads or known ligand structures, under the form of fingerprints, following the same procedure as herebefore described, except for the fact that the used conformers are those directly generated by running the "Catalyst" Software of MSI, to which the sketches of these reference compounds are submitted as such;
- introducing a scoring function, in step 16, which successively compares the distance distributions corresponding to each pair of features (fa, fb).

First, the 21 partial scores expressed as pnorm1(fa, fb), pnorm2(fa,fb) and pcross(fa,fb), are calculated in the form of convolution products for every pair of features, as follows

$$pnorml(fa,fb)= \sum_{i,j=1...12} FP\_mol1(fa,fb,i)*FP\_mol1(fa,fb,j)*e^{-\alpha(i-j)*(i-j)}$$

$$pnorm2(fa,fb)= \sum_{i,j=1...12} FP\_mol2(fa,fb,i)*FP\_mol2(fa,fb,j)*e^{-\alpha(i-j)*(i-j)}$$

$$pcross(fa,fb)= \sum_{i,j=1...12} FP\_mol1(fa,fb,i)*FP\_mol2(fa,fb,j)*e^{-\alpha(i-j)*(i-j)}$$

where FP_moll and FP_mol2 are the fingerprints of the first compound, a reference one, and, respectively, the second compound, a tested one, i and j are variables looping over all the considered distance bins, as described at step 11, and $\alpha$ an exponential damping factor.

If pnorml(fa,fb) and pnorm2(fa,fb) are simultaneously zero, it means that the corresponding pairs of features do not occur in any one of the molecules; therefore, such combinations are ignored when evaluating the global similarity score between moll and mo12. Otherwise, the partial similarity score per feature pair, psim(fa,fb) is defined by :

$$psim(fa,fb)=2pcross(fa,fb)/[pnorm1(fa,fb)+pnorm2(fa,fb)]$$

And the similarity score by a sim-score which involves a weighting factor:

$$\text{sim-score}=1-[\Sigma\ W(fa)W(fb)psim(fa,fb)]/[\Sigma\ W(fa)W(fb)]$$

where W(f) is the weighting factor for the feature pairs (fa, fb).

In the sim-score expression, both sums are taken over the feature pairs (fa,fb) for which at least one of pnorml (fa,fb) and pnorm2(fa,fb) are not zero, e.g. the pairs that appear in at least one of the two molecules.

The weighting factors W(f) are the tunable parameters of the method together with the exponential damping factor that controls the value of pnorml, pnorm2, pcross. The values of such tunable parameters are obtained by different calibration approaches, aimed to optimize the overall performance of the model.

- in step 17, all the molecules mol2 of the potential library for which their similarity score, sim-score, with respect to the reference compound moll is less than a specified threshold are listed in order of increasing sim-score value. The approach can now continue with the synthesis and testing of the compounds displaying the best scoring fingerprints, or alternatively, the structures output by the fast fingerprint screening method can be submitted to alternative, more precise, but much more time-consuming similarity-score evaluation schemes, too slow to be applied for the direct scoring of all the compounds in the potential library, but adapted to handle a set of typically 1000-10000 compounds retrieved after elimination of the compounds with no matching fingerprints. This coupling of a high-throughput scoring method based on fingerprints and a slow, but more accurate superimposition procedure combines the advantage of the rapidity of the former and the accuracy of the latter, in order to retrieve the best matching candidates out of the potential library. In a step 18, the candidates retrieved on the basis of a sufficiently high fingerprint similarity with respect to the reference compound, are submitted to a more elaborate evaluation of the diversity score, by a "ComPharm" superposition algorithm attempting to superimpose each of the candidate compounds on the reference molecule, such as to maximize the overlap of the pharmacophoric centers of the former with pharmacophoric centers of the same kind of the latter. This methodology is similar to the previously cited CoMFA approach in that it tries to find an optimal superposition of two compounds, but focuses on pharmacophoric features rather than on global molecular properties such as the steric or electrostatic fields.

In a step 19, the ComPharm module retrieves the multiconformational models of the reference compound (comprising the conformers $R_1,R_2,...R_C$) and the candidate compound (set of conformers $C_1,C_2,...,C_C$) from the ghosts database mechanism, together with the list of pharmacophoric feature lists, in the same way this was done by the fingerprint evaluation mechanism (see steps 11-12).

In a step 20, a ComPharm similarity score is defined such as to measure the global degree of spatial overlap between the atoms that possess pharmacophoric features in the candidate and the corresponding atoms carriers of the same features in the reference molecule. Partial overlap scores per features pscore-ovrl(R,C,f) measure the degree of overlap for each of the previously considered pharmacophoric features f1-f6, for example the partial score of overlap between the hydrophobic groups in the candidate and the hydrophobic groups in the reference; between the positive charges in the candidate and the positive charges in the reference, etc.

pscore_ovrl(R,C,f)sum over {i=atoms of R having feature f} (sum over {j=atoms of C having feature f} (exp($-\alpha$*dist(i,j)$^2$)))

where dist(i,j) represents the euclidian distance between atoms i and j in the current relative orientations of the molecules. Based on the definition of pscore_ovrl, partial similarity scores are defined in the following two different manners :

pscore_sim_strict(R,C,f)=2*pscore_ovrl(R,C,f)/[(pscore_ovrl(R,R,f)+pscore_ovrl(C,C,f)]

pscore_sim_match{R,C,f)=pscore_ovrl(R,C,f)/ score_ovrl(R,R,f)

The strict similarity measure pscore_sim_strict reaches 1 only if R and C are identical. However, pscore_sim_match only measure what fraction of the features present in the reference will be matched by the candidate. If the candidate C includes R as a substructure, but has an arbitrary number of supplementary functional groups that are not present in R, pscore_sim_match will nevertheless reach 1, since all the features present in R are also present in C. In choosing pscore_sim_match rather than pscore_sim_strict to calculate the ComPharm similarity score, the user will penalize a candidate molecule for failing to present the required number of pharmacophoric centers overlapped on those of the reference, but not for introducing new features no present in the reference. With pscore_sim_strict, the candidate is expected to display neither too few nor too many pharmacophoric features, oriented as required to match those of the reference.

As with the fingerprint overall score, the ComPharm overall score will be taken as a weighted average over all the considered features f of the pscore_sim terms, ignoring those features for which the denominators in the expressions of the latter are zero. The corresponding weighting factors W(f) and the damping factor alpha in the above exponential are the ones previously used by the fingerprint scoring function.

In a step 21, a ComPharm optimizer engine will use a genetic algorithm in order to find a pair of conformers (Ri,Cj) and the relative orientation in which they have to be brought in order to maximize the ComPharm overall score, as previously defined. This maximal ComPharm score will now be used to rerank the candidates retained after the fingerprint comparison step.

In a following step, the chemical synthesis of the best ranking compounds will be undertaken.

Alternatively or cumulatively, a list of all the building blocks represented in the retrieved products is established and a generation focused combinatorial library is based on such BBs.

The values of the weighting factors may be obtained by two possible approaches according to the specific search to be carried out, the General Diversity paradigm and the Receptor-Oriented paradigm.

The General Diversity paradigm consists in choosing the weighting factors in order to obtain a similarity scoring function which successively discriminates between classes of ligands of different receptors. Given an arbitrary collection of clusters of ligands associated to different receptors, each cluster consisting of families of ligands that exclusively bind to the associated receptor, the «most diverse» subset of ligands retrieves one ligand per receptor if the scoring function on which this most diverse subset selection has been realized has an ideal discriminative power. Because a less discriminating function would select several ligands of the same receptor, while completely ignoring other ligand families, the weighting are optimized in order to improve the discriminative power of the distances between two molecules by using as an objective function the number of receptors for which at least one ligand has been picked out in the most diverse selection. Then the obtained weighting factors values characterize, if the number of receptors is sufficiently large, the average propensities of the bipolar pharmacophores to contribute to the anchoring of a ligand in a receptor site.

The Receptor-Oriented Diversity paradigm consists in calibrating the weighting factors on the basis of primary screening results of a library against a given receptor, such as to minimize the average distance between any two active compounds and to maximize the dissimilarity scores between each active and any inactive compound. This calibration mode allows to define which pharmacophores are essential for the binding to a given receptor.

Also, the screening of the ghost library can be performed by Recursive Partitioning, Neural Networks or other Quantitative structure-Activity Relationships, instead of similarity scoring, provided that sufficient information is available to perform the data-mining required for the calibration of the former methods. While similarity scoring has the advantage of requiring a single active compound to produce analogs thereof, it has the drawback of no knowledge concerning the molecular features that are the most important for the expected activity and therefore indiscriminately imposes that all (both relevant and irrelevant features of the lead) have to be present in the retrieved analogs.

Recursive Partitioning is a data mining method which, starting from a large set of experimental measures of an observable (the biological activity) establishes a clustering scheme with respect to a set of variables that determine the outcome of this experiment (molecular descriptors, i.e., the pharmacophoric fingerprints). The goal of the approach is to construct clusters that are homogeneous with respect to the value of the observable, which should fall within a same range (same class) for all the data points grouped together in a cluster. An input table specifying the activity class of each compound associated to the molecular descriptors characterizing that molecule is be provided in order to assign each compound to an "activity cluster" in function of the established "activity rules" and of the values of its representative descriptors. The program will find what the "most representative descriptors" are and what the threshold values of these descriptors define the belonging of a compound to one or the other family. The optimal "splitting rules" will define a "decision tree" in which the individual families are optimally enriched in "active" and respectively "inactive" compounds.

According to a set of statistical rules, the algorithm selects which descriptors should be used and which threshold values to be taken in order to make the split. The molecules are then assigned to the corresponding "leafs" of the decision tree. A leaf of class 1 should ideally contain only active molecules. However, this is highly unlikely to happen in real life. Typically, each leaf contains both actives and inactives. If the relative ratio (Nr. actives/Nr. Inactives)$_{leaf}$ is larger than (Nr. Actives/Nr. Inactives) $_{total\ population}$, then that leaf will be considered to be a leaf of class 1. The error committed when predicting the inactives in this leaf to be active is considered to be smaller than the one introduced if that leaf would have been labeled "class 2" and the actives contained in it would have been predicted inactives. The opposite is true for a leaf in which the relative concentration of actives falls below their concentration in the full set of molecules. In conclusion, leafs of class 1 are leafs that are enriched, whereas leafs of class 2 are impoverished in actives.

This "data mining" approach thus extracts a set of features (pharmacophoric fingerprints) which appear more often among the active compounds in the learning set than in their inactive counterparts. The approach singles out the compounds that display the "important" features and "predict" that they should be active.

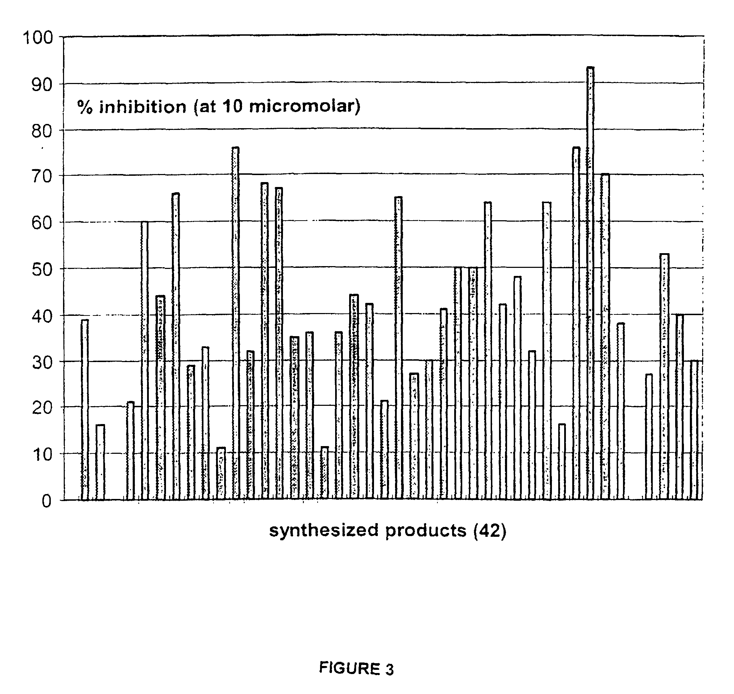

In order to illustrate the General Diversity paradigm, six reference ligands of the DAT receptors (dopamine carrier / IDM) have been used to carry out a first proof-of-concept study. Compounds of different chemistries, chemistry of functional rearrangements, reductive amination, amides, urea, carbamates and esters, have been sorted according to the filters and the general diversity paradigm according to the present method. This virtual screening approach suggested the synthesis of a combinatorial library of amides issued from 2 acids x 21 amines from a reductive amination of aromatic aldehydes. This small library of 42 compounds has been synthesized and tested, with very positive results, which are illustrated in Figure 3.

These compounds are novel, quite different from the point of view of atomic connectivity and certainly belong to an "open" class of amides which can be easily synthesized and further optimized by means of focused libraries. The virtual screening approach therefore successfully discovered analogs, only 10 times less potent than the reference molecules, but displaying an obvious and well known retrosynthetic route.

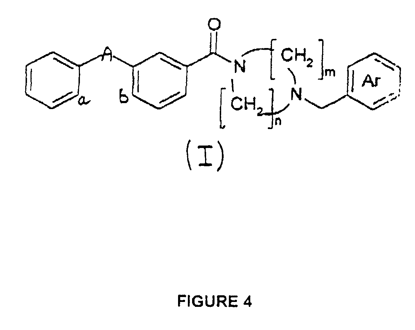

In this respect, the invention also discloses compounds of general formula (I) represented in Figure 4, in which A represents C=O, C=S or CH-OH ; n and m, independently from one another, are 1, 2 or 3, Ar is an aromatic group, preferably selected from fluorene, benzylstyrene, 4,4'-biphenyle, phenanthrene, phenyle, 4-chlorophenyl and 4-bromophenyl, and in which the carbon atoms a and b can be bound to each other. More preferably, A represents C=O. Still more preferably, in the general formula (I), n and m are 2. Most preferred compounds are represented by the generale formula (I) in which A is C=O, n and m are 2, and the carbon atoms a and b are not bound.

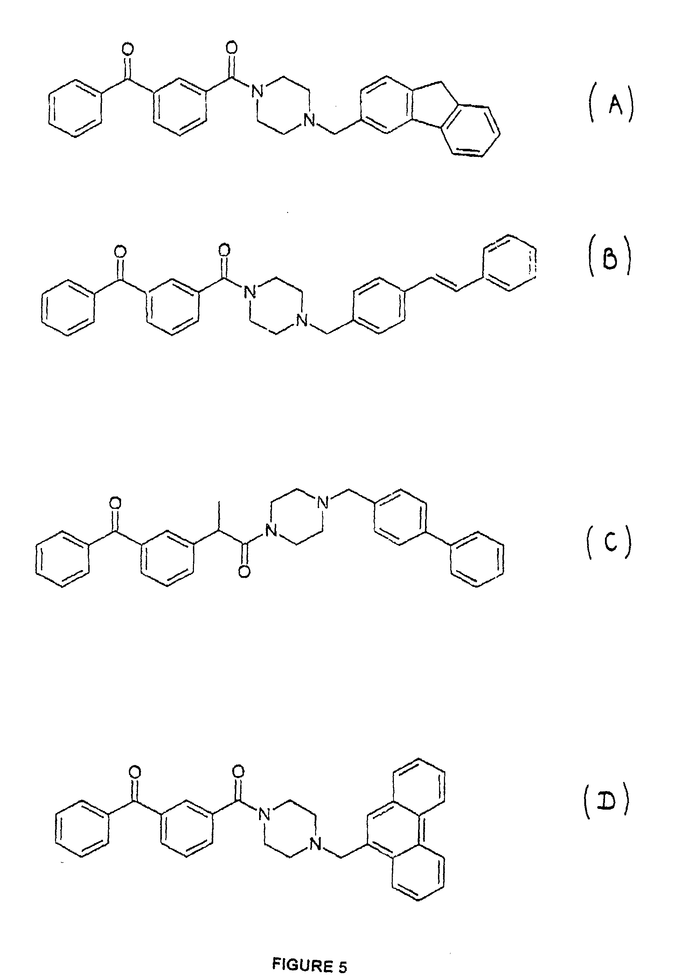

Some of the most active compounds, namely compounds (A), (B), (C) and (D), are represented in Figure 5. These compounds possess advantageous biological and structural properties for use in the pharmaceutical and/or agrochemical industry. In particular, said compounds can be used for inhibiting the binding of ligands to the DAT receptor, in vitro or in vivo, for the production of analogs thereof bearing different substituting groups as well as for the production of library of compounds. The invention thus also discloses library of compounds comprising at least one, preferably a plurality of compounds as defined above.

As indicated above, these compounds can be easily synthesized according to conventional chemical techniques known to those skilled in the art. In particular, they can be prepared from the precursor amine, as illustrated in the experimental section.

According to another example, analogs to the serotonine activity on the $5\text{-HT}_4$ receptor (ability of compounds to inhibit $5\text{-HT}_4$ induced contractions of guinea-pig ileum preparations) have been retrieved according to the general-diversity scoring function of the invention. More specifically, the application of the present method to the generation of analogs of a lead compound active against $5\text{HT}_4$ receptors has been tested.

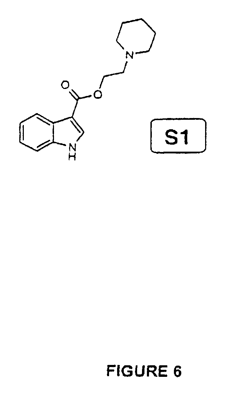

In this example, the fingerprint of compound S1 (shown in Figure 6) has been produced by conformational sampling according to the method of this invention, and a subset of 5 million fingerprints of the potential fingerprint library has been screened to retrieve the compounds with best scoring values.

Starting from the $5\text{HT}_4$ ligand S1, the virtual screening approach retrieved :

- a series of 60 carbamates. Their constituting building blocks (11 alcohols and 28 amines) combined to yield a small combinatorial library of 7 alcohols x 15 amines = 105 carbamates, since 4 alcohols and 13 amines had to be discarded due to availability and reactivity problems.
- a set of 35 esters, which would have been circumscribed by a library of 8 acids x 7 alcohols = 56 compounds. Only the 8 best ranking esters, including the reference compounds itself, found to be a member of the screened subset of the fingerprint library, have been synthesized.

These compounds have been screened on the 5HT4 receptor target to determine their binding ability, and thus verify the predictability of the present method. Table 1 outlines the results of the biological tests for all the 8 synthesized esters and the 8 most active carbamates.

Table 1

| NO. | PRODUCT | % INH (10 $\mu$M) | IC$_{50}$ (NM) | TYPE |
|---|---|---|---|---|
| 1 | S1 | 104 | ** | ester |
| 2* | %33518 | 101 | ** | ester |
| 3* | %33519 | 104 | ** | ester |
| 4* | %33520 | 104 | ** | ester |
| 5* | %33521 | 103 | ** | ester |
| 6 | %33522 | 101 | ** | ester |
| 7* | %33524 | 103 | ** | ester |
| 8* | %33539 | 9 | Inact. | ester |
| 9* | %21910 | 65,8 | 9270 | carbamate |
| 10* | %21947 | 61,8 | 4010 | carbamate |
| 11 | %21954 | 51,3 | 7360 | carbamate |

Active compounds - * displays the actually predicted molecules.

** These esters have affinities of the same order than of magnitude than the reference compound, e.g., in the nanomolar range.

Table 1   (continued)

| NO. | PRODUCT | % INH (10 μM) | IC$_{50}$ (NM) | TYPE |
|---|---|---|---|---|
| 12 | %21961 | 49,2 | 32400 | carbamate |
| 13 | %21968 | 57,1 | 11070 | carbamate |
| 14 | %21975 | 64,3 | 8460 | carbamate |
| 15* | %21984 | 64,4 | Inact. | carbamate |
| 16 | %21989 | 64,6 | 5490 | carbamate |

Active compounds - * displays the actually predicted molecules.

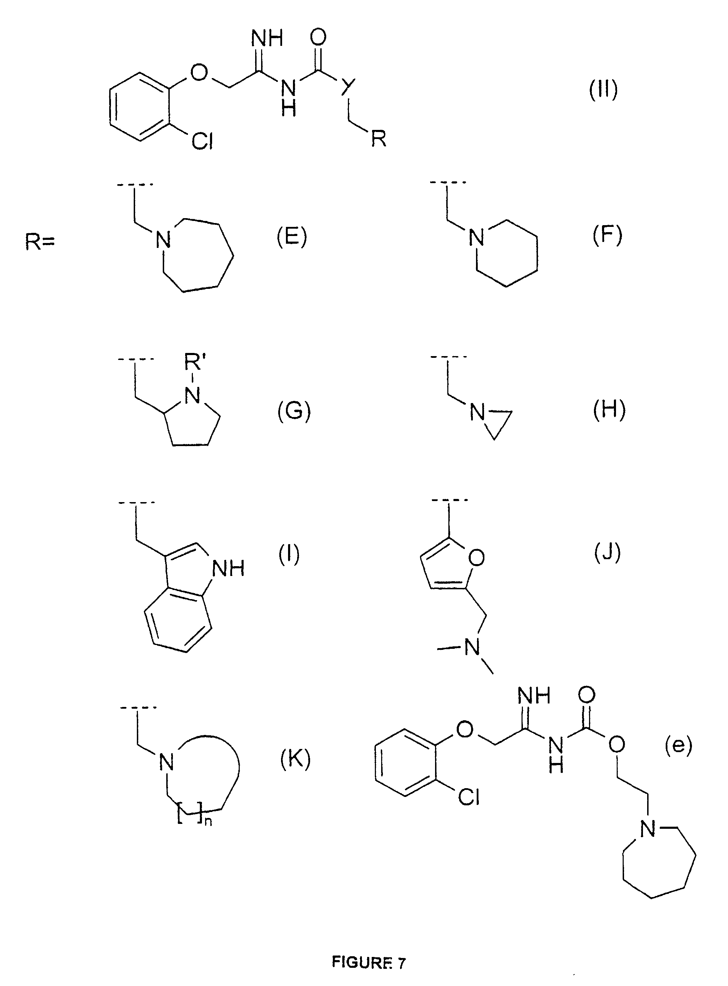

In this regard, the invention also discloses novel (chlorophenoxy) compounds having an original structure and advantageous biological properties. Said compounds are represented by the general formula (II) shown in Figure 7, in which Y is O, N or CH2 and the substituent R comprises a heterocyclic group. In a particular embodiment, the substituent R comprises a heterocyclic group selected from the groups (G), (I), (J) or (K), in which n is 1, 2, 3, 4 or 5 and R' is an alkyl, preferably a lower alkyl (having between 1 to 5 carbon atoms), more preferably a methyl or ethyl group. Particular examples of group (K) are groups (E), (F) and (H). More preferably, in the general formula (II), Y is O and the substituent R comprises a heterocyclic group. Even more preferably, Y is O and the substituent R is represented by the formula (E) to (K) shown in Figure 7, in which n is 1, 2, 3, 4 or 5 and R' is an alkyl, as defined above.

Specific compounds are compound (e) which is represented on Figure 7, and which corresponds to the general formula II wherein Y is O and R is (E); compound (f), which corresponds to the general formula II wherein Y is O and R is (F); compound (g), which corresponds to the general formula II wherein Y is O and R is (G); compound (h), which corresponds to the general formula II wherein Y is O and R is (H); compound (i), which corresponds to the general formula II wherein Y is O and R is (I) and compound (j), which corresponds to the general formula II wherein Y is O and R is (J).

These compounds possess advantageous biological and structural properties for use in the pharmaceutical and/or agrochemical industry. In particular, said compounds can be used for inhibiting the binding of ligands to the serotonin receptor 5HT4, in vitro or in vivo, for the production of analogs thereof bearing different substituting groups as well as for the production of library of compounds. The invention thus also discloses library of compounds comprising at least one, preferably a plurality of compounds as defined above.

As indicated above, these compounds can be easily synthesized according to conventional chemical techniques known to those skilled in the art. In particular, they can be prepared from the precursor chlorophenoxy compound, in the presence of carbonyldiimidazole and the heterocyclic group, as illustrated in the experimental section.

The invention also discloses compositions comprising the compounds of formula (I) and/or (II) disclosed above and a vehicle, for instance a pharmaceutically acceptable vehicule (a saline, isotonic and/or physiologic solute, a gel, a surfactant and/or a stabilizing agent, etc.). Said compositions can be formulated in any appropriate device.

These results show that the virtual screening approach of the present invention is able to:

- successfully find the nearest neighbors of a compound out of a collection of 5 million molecules. If very similar "me too" compounds exist in the virtual library, the method will almost certainly retrieve them. Actually, the reference ligand itself was a member of that virtual library and has been "rediscovered" by the virtual screening engine. While this may appear trivial, it is in fact a validation of the way in which the fingerprints were built on hand of multiconformational models. The reference fingerprint of S1 was generated on the basis of conformers of S1 directly provided by the Catalyst program, while the one stored in the potential fingerprint library was based on ghost-database conformers. Due to the "fuzzy" definition of the fingerprints and the definition of the similarity scoring function, these fingerprints remain highly similar in spite of the differences in the conformational search approaches at the basis of their construction.

- identify and define a different family of ligand -carbamates-, which, although not always as potent as the reference compound, are much easily available through combinatorial synthesis and represent a class on which further optimization work can be done by means of focused libraries. While the overall pharmacophoric distribution in the carbamates and in the original ester are highly similar and the atom connectivity is still fairly well preserved (e.g. the indole ring is conserved in all the active carbamates), this is nevertheless a distinct, novel class of 5HT$_4$ ligands.

- ensure an important hit rate among the compounds that are actually predicted - the virtual screening "hits", with respect to the rest of the compounds in the combinatorial matrix synthesized in order to circumscribe these hits. Out of the 14 active compounds, 7 were virtual screening hits. The virtual screening had predicted that 17 molecules should have been active and 7 actually did - this is a very good success rate for such a simple pattern recognition algorithm.

As another validation of the present screening and analoging method, a biased library of 6400 compounds, focused around of a BZDc-receptor hit obtained from a primary screening of a lead seeking library. Since the lead found in the lead seeking library were already combinatorial products of the type A-B, this biased library was obtained by retrieving, using the FBPA-similarity scoring approach with the "general diversity" parameters, sets of similar analogs $A'_1$ of the building block A, plus sets of similar analogs $B'_1$ of the building block B, and then synthesized all the 6400 resulting products $A'_1-B'_1$ (left-right analoging strategy). This biased library was then submitted to high-throughput screening against the BZDc receptor, at the same concentration of $10^{-5}$ mol/l used to screen the initial, lead-seeking library. As shown in the following table 2, a sensible increase in the hit rate of the focused library with respect to the lead-seeking library can be evidenced. Furthermore, the biased library is specifically enriched in highly active compounds, which occur 5 times more often in the biased library, while representing very rare event in the lead seeking library.

TABLE 2

| % OF INHIBITION at 10-5 M | LEAD-SEEKING LIBRARY | | FOCUSED LIBRARY | | ENRICHMENT FACTORS OF THE HIT-RATE |
|---|---|---|---|---|---|
| | # of Hits | Hit rate | # of Hits | Hit rate | |
| $\geq 50\%$ | 30 | 1.63 | 221 | 4.85 | 2.98 |
| $\geq 70\%$ | 12 | 0.65 | 82 | 1.8 | 2.77 |
| $\geq 80\%$ | 6 | 0.33 | 67 | 1.47 | 4.45 |
| $\geq 90\%$ | 4 | 0.22 | 53 | 1.16 | 5.27 |

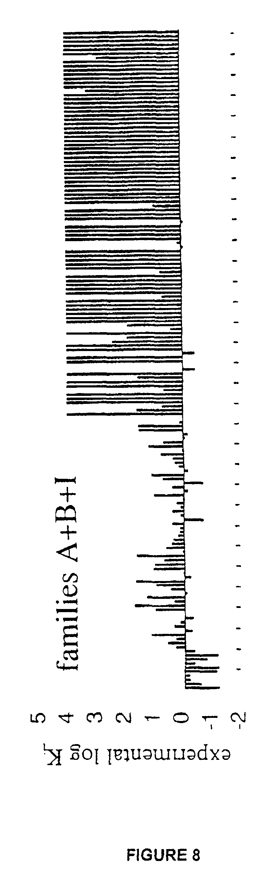

Several other validations tests of the Fuzzy Bipolar Pharmacophore Autocorellogram (FBPA) metric have been performed and one of the most relevant ones consisted in discriminating between ligands of farnesyl-protein-transferase (FPT). Using the most active farnesyl-protein-transferase (FPT) and inactive compounds. Using the most active FPT inhibitor as a reference ligand, the method of this invention successfully discriminated between active compounds (consisting of a class A of inhibitors that shared a common skeleton with the reference plus a structurally distinct class B) and inactives I (Figure 8). In particular, the results presented in Figure 8 show that the similarity scores obtained indeed correlated with the activity of the molecules, i.e., that the Fingerprints produced can allow the production of focused libraries of compounds with increased hit rates.

The recursive partitioning approach has been validated on hand of 15025 compounds previously analyzed for activity against the mu receptor. The chemist's classification of compounds into "hits" and "inactives" has been adopted as such. In this study, all the selected "hits" were assigned into a class "1", while the inactives were labeled as members of the class "2". According to this classification, 67 compounds out of the 15025 (0.445%) were active molecules. Obviously, this classification into actives and inactives is artificial and, while we can be pretty confident that the compounds in class 1 are indeed $\mu$ ligands (they cover a wide range of activity: from nanomolar to micromolar IC50 values), some of the molecules that are input as examples of inactive compounds to the model are actually active as well. This is an unavoidable source of error of any model bound to interpret raw HTS data, unless huge effort is spent to characterize every compound of the library in terms of precise IC50 measurements. The model must be robust enough in order to accommodate a certain fraction of "false negatives" without a serious disruption of its learning process. The success parameters of the model in classifying a compound as active or inactive may be somehow underestimated due to this problem. Classifying a "false negative" as active counts as an error from the point of the view of the model, but is actually none.

The totality of 15025 molecules was then split into 3 different learning sets $LS_1$, $LS_2$, $LS_3$ containing each 22, 22 and respectively 23 active compounds, dispatched such as to ensure a homogeneous representation of the main families of actives in each one of them. Each LS contains representatives of morphine derivatives, amides, sulfonates and carbamates.

The pharmacophoric fingerprints of active and inactive molecules were analyzed as disclosed before and a decision tree was defined. The RP decision tree built on hand of the learning set $LS_1$ has then been used to predict the actives in sets $LS_2$ and $LS_3$ while the tree calibrated on $LS_2$ was used to search for actives in $LS_1$, and $LS_3$. The average quality criteria of these test runs are given in the table below. As expected, the success rates on sets that were not used for calibration are still significant, while much lower than those obtained on the calibration sets.

| QUALITY CRITERIA | TEST SETS | LEARNING SETS |
|---|---|---|
| %DA | 36.67% | 95.45% |
| %HD | 7.86% | 19.81% |

(continued)

| QUALITY CRITERIA | TEST SETS | LEARNING SETS |
|---|---|---|
| EF | 17.6 | 46.4 |

%DA : The fraction of discovered actives, e.g., the fraction of actives that a user would discover when screening only the subset of compounds predicted by this algorithm, with respect to the whole number of actives he would obtain if taking the effort to screen the complete set of available compounds.

%HD : The hit density, representing the relative fraction made up by actives among the subset of compounds predicted to be active by the algorithm.

EF : The enrichment factor, representing the ratio between the high density in the enriched subset of compounds predicted to be active, with respect to the "blind" hit rate in the complete set of available compounds/

The above disclosed RP trees' method based on fingerprints of this invention was compared to RP trees based on standard topological and shape descriptors. For that purpose, alternative RP trees were constructed on hand of the same learning sets, using the standard descriptors set available under Cerius2 - including shape, structural and topological descriptors, as well as the total charge of the molecule and the AlogP value. Their predictive powers on the testing sets not used for calibration is given in the following table:

| Quality criteria | Fingerprints | Standard descriptors |
|---|---|---|
| %DA | 36.67% | 27.77% |
| %HD | 7.86% | 7.46% |
| Ef | 17.6 | 16.7 |

The most significant advantage brought by the fingerprints of the present invention consists in an improvement of 9% of the capacity to recognize "foreign" actives, not used for calibration. The most likely explanation of this difference is that compounds not used at the calibration step, which are displaying a similar pharmacophoric pattern, but a different topology with respect to some compounds used at calibration, are more likely to be recognized as "actives" when using fingerprints. A difference in topology may have important consequences on the 2D-descriptor values, without affecting the pharmacophoric pattern and the activity, and therefore cause the misclassification of that molecule by a 2D-descriptor-based, but not by the FBPA-based decision tree. Besides, another important advantage in working with Fingerprints according to this invention resides in the much smaller effort required to evaluate them. The generation of Fingerprints files for the 15025 molecules took roughly 15 minutes, while 18 hours were necessary to complete the standard descriptor calculations (on the same R10000 CPU).

The invention is not limited to the examples as described and illustrated, In particular, different reference libraries corresponding to different chemistries can supply the reference library of the Bbs. In other respects, the present invention can be applied to different contexts, for instance to search analogs to a given product having specified chemical properties (a detergent, ...).

Experimental Section : Protocols for the synthesis of the compounds of formula (I) and (II).

1. Synthesis of compounds (I)

This example specifically illustrates the synthesis of compound (A) represented in Figure 5. The protocols and methodologies described can be easily applied to the synthesis of any compound of general structure (I) by the skilled artisan, using common general knowledge.

1.1. Synthesis of the precursor amine

In a 50 mL balloon, under magnetic steering, 337.16 mg of TBTU (1.05 mmol; 1.15 eq.) are added, and dissolved in 3.89 mL DMF to obtain a 0.27 M solution. 205.96 mg of 3-benzoylbenzoic acid (0.91 mmol; 1 eq.) are added, to which 215.4 µL DIEA (1.75 mmol; 19 eq.) are added. The solution is steered for 3 minutes. 156.24 mg N-Boc-piperazine (0.84 mmol; 0.9 eq.) are the added, and the solution is steered overnight at room temperature. The solution is concentrated by vacuum and the resulting oil is suspended in 3 mL methanol. The solution is steered for 10 minutes, and 10 mL $Na_2CO_3$ are then added. An extraction is then performed with 10 mL DCM, twice. The organic phases are pooled and washed once in 10 mL $Na_2CO_3$ 1M, twice in 10 mL HCl 1M, and once in 10 mL distilled water. The organic phase is dried on $MgSO_4$ and the solvent is evaporated under vacuum.

A brown oil (368 mg) is thereby obtained. It is suspended in 1.68 mL of a TFA / DCM solution (50/50), and steered for 1 hour at room temperature. The solvent is evaporated under vacuum and the resulting oil is suspended in 3 mL acetonitrile. The solution is placed in a dryer. 279 mg (0.68 mmol; yield: 81.6%) of the expected precursor amine are obtained in the form of a yellow oil.

1.2. Synthesis of compound (A)

In a deep-well, 22 µL of a 0.25 solution of the precursor amine in DCM (5.5 µmol; 1.1 eq.) are introduced, together with 50 µl of a 0.1 M (5 µmol; 1 eq.) solution of 3-fluorenecarboxaldehyde in DCM. The deep-well is steered at room temperature for 5 minutes. Then, 25µl of a suspension of Tris Sodium acetoxyborohydrate 0.5 M (12.5 µmol; 2.5 eq.) in DCM are added and the deep-well is steered overnight at room temperature. The solvent is evaporated and the resulting oil is suspended in 100 µl DCM. The hydrate excess is neutralized by 15 µl $NaHCO_3$ 1 M (3 eq.) and the deep well is strongly steered. The solvents are evaporated and compound (A) is obtained.

This protocol can easily be transposed to other compounds of general structure (I) having different aromatic groups, by using the corresponding aldehyde. Also, the precise conditions can be adapted by the skilled artisan.

2. Synthesis of compounds (II)

This example specifically illustrates the synthesis of compound (e) represented in Figure 7. The protocols and methodologies described can be easily applied to the synthesis of any compound of general structure (II) by the skilled artisan, using common general knowledge.

In a deep-well, 50 µl of a 0.1 M solution of 2-(hexamethylene imino) ethanol in DMF (5 µmol; 1 eq.) and 42 µl of a 0.247 M solution of N, N'-carbonyldiimidazole in THF (10.3 µmol ; 2.1 eq.) are introduced. The deep-well is steered at room temperature for 2 hours. 50 µl of a 0.1 M solution of 2-(2-chlorophenoxy) acetamidinium chloride in DMF (5 µmol; 1 eq.) are then added. The deep-well is steered at room temperature for 12 hours; and compound (e) is obtained.

**Claims**

1. A method for rapidly retrieving similar, and therefore potentially active synthetic analogs of a lead compound from a reference library of building block compounds and from synthesis protocols for the combinatorial synthesis of the said analogs, the said method comprising the steps of:

   - selecting building blocks in the reference library by a chemical filter algorithm comprising elementary chemical rules and, optionally, reactivity prediction models based on specific reactivity descriptors, the selected building blocks being valid reaction partners in the chemical processes of the combinatorial synthesis, detecting their reactive centers and converting their molecular sketches into radicals, said radicals being the substructures that make up the combinatorial products;
   - building 3D-multiconformational models of these radicals by linking them temporarily in a 2D-sketch to a bulky spacekeeper group and submitting the resulting complex to a conformational sampling run yielding a collection of 3D conformers, then removing the spacekeeper in order to ensure that the reactive centers of the conformers are sterically accessible and their free valencies points towards a region of free space previously occupied by the spacekeeper;
   - registering these 3D-conformers, after verifying that they comply with certain sterical hindrance and conformational diversity criteria, into a 3D-radical database that serves as an input for a "ghost database" of combinatorial products, the "ghost database" comprising an algorithm generating a database of combinatorial products by linking together the registered conformers of the constituting radicals;
   - for any molecular structure that is accessible within the ghost database, detecting any atom that displays physical property features of the pharmacophoric type on the basis of elementary rules accounting for the chemical nature of such an atom and the molecular environment in which it is placed, these pharmacophoric features being involved in determining the intensity of intermolecular interactions and comprising at least some of the hydrophobic, aromatic, hydrogen bond donor, hydrogen bond acceptor, anionic and cationic characters, and listing all the possible pairs of pharmacophoric features in which each atom displays one of said pharmacophoric features;
   - for each pair of pharmacophoric features detected in the said molecular structure, calculating all the distances between the involved atoms in every conformation of this molecule, assigning in a fuzzy manner said calculated distances to a plurality of distance classes and creating a distance distribution density;
- generating a conformational fingerprint vector that contains the distance distribution densities of all the pairs of pharmacophoric features associated to a current conformation of the molecule, calculating an average molecular fingerprint from the conformational fingerprints of all the considered conformations and registering this average molecular fingerprint to constitute and supply a potential library,
- defining a scoring function to evaluate a measure of similarity between two molecular fingerprints, accounting for the relative importance of the pharmacophoric features owing to weighting factors that are calibrated in order to maximize a discriminative power with respect to different binding affinities; and
- generating the fingerprints of the lead compound, comparing these fingerprints to each molecular fingerprint of the potential library according to the above scoring function and listing in order of increasing score values the molecules of the potential library for which the scoring function gives score values less than a specified threshold.

2. A method according to claim 1, **characterized in that** the weighting factors of the scoring function are calibrated in order to maximize the discriminative power between families of ligands of different receptors in a « General Diversity paradigm » by using as an objective function the number of receptors for which at least one ligand has been picked out in the most diverse selection.

3. A method according to claim 1, **characterized in that** the weighting factors of the scoring function are calibrated in order to maximize the discriminative power between the compounds that bind to a given receptor, in contrast to those that have no binding affinity with respect to it, in a « Receptor-Oriented Diversity paradigm » by primary screening results of a library against a given receptor, such as to minimize the average distance between any two active compounds and to maximize the dissimilarity scores between each active and any inactive compound.

4. A method according to anyone of the preceding claims, **characterized in that** preliminary checking steps in order to discard current building blocks which could include potentially interfering reactive groups and/or to add functional groups to a building block prior coupling are included.

5. A method according to anyone of the preceding claims, **characterized in that** the yielding of 3D conformers is completed with an encoding structural information step over a geometrical filter, by extracting from a conformational fast sampling analysis of the molecular structures of 2D-sketches of the selected radicals in connection with pharmacophoric features and with respect to each specific chemical group involved in each radical to detect and discard remaining conformations of important steric hindrance around the reactive center and conformations similar to other sampled ones.

6. A method according to anyone of the preceding claims, **characterized in that** the building blocks currently available from a reference library and a set of synthesis protocols are regularly updated, each such update automatically triggering the update of the potential libraries.

7. A method according to claim 1, **characterized in that** it is integrated to a discovery paradigm defining a feedback loop starting from a primary, "blind" screening of a compound library, and including the steps of identification of active compounds, training and adjusting parameters of a predictive model to recognize the specific features of active compounds, retrieval of potentially active analogs for these compounds out of a reference library according to the instant method, synthesis of these analogs and screening to refine the parameters.

8. A method according to anyone of the preceding claims, **characterized in that** it comprises essentially the following steps:

- scanning a reactivity profile of the current synthesis consisting of required groups and interfering groups (step 1);
- checking if the selected building block contains a single molecule and deleting the counterions (step 2);
- checking the presence of any interfering groups in the selected building block and discarding such current building block (step 3);
- checking the presence of required functional groups in the building block and figuring out the reactive center involved in the bond with a second building block to form reactive partner radicals (S1, S2), eliminating the leaving groups (step 4);
- adding hydrogens to the heavy atom skeleton of the sketches of each radical (S1, S2), at least six pharmacophoric features, aromaticity, hydrophobicity, hydrogen bond donor or acceptor property, positive and negative charge of every atom, being checked and listed (step 6);
- anchoring the 2D-sketch of the radical with its free valency to the bulky spacekeeper group (step 7);
- submitting the resulting 2D-sketch of this compound to a known conformational sampling run, yielding a collection of conformers (step 8);
- severing and deleting, for each of the conformers obtained (step 8), the spacekeeper moiety, restoring the free valency of the radical which now points towards the empty region previously occupied by the spacekeeper (step 9);
- performing, for each retained conformer, a coordinate transformation in a reference system (OXYZ), in order to place the reactive center in the origin of the reference system and to align the free valency along the Z-axis (step 9);
- generating a list of all the compounds that are obtained by coupling each radical (S1) with every one of its partners (S2) (step 10);
- looping over all pairs listed (step 10) and storing current pairs of radicals (S1, S2) together with all their available conformers, as obtained (step 9) and the lists of pharmacophoric features of the composing atoms formerly used, the pharmacophoric features of the atoms linked by new bonds being reevaluated (step 11)
- linking each conformer (S1i) of the first radical (S1) with each conformer (S2j) of the second radical (S2) in mirroring the coordinates of the latter with respect to the XOY plane, translating them along an axis (Z) in order to restore the correct length of the newly formed bond between the conformers ; and rotating around the new axis (step 12);
- in order to construct the distance distribution density of pairs of pharmacophoric features, evaluating a complete set of interatomic distances for the current conformation of each dimer; all the distances between pairs of atoms which match a given pair of pharmacophoric features being classified into distance classes (step 13);
- representing a generated conformational fingerprint vector describing the current geometry with a number of elements with respect to the number of combinations that can be obtained with the pharmacophoric features, each fingerprint element (FP) being equal to the number of atoms pairs matching a given pair of pharmacophoric features (fa,fb) and which are separated by a distance which falls within an above-mentioned distance class (step 14);
- summing the conformational fingerprints to obtain a molecular fingerprint vector, and norming this sum with respect to the numbers of considered conformers of the product to obtain an average molecular fingerprint which is stored in association with the identification codes of the radicals which compose that product; the collection of all the fingerprints of all the possible coupling products between all building blocks qualifying the initial synthesis processes and defining potential libraries. (step 14);
- encoding of leads or known ligand structures, under the form of fingerprints, following the above procedure (step 14), but using the conformers gielded by said known conformational sampling run (step 8) to which the sketches of these reference compounds are submitted as such (step 15);
- introducing a scoring function, which successively compares the distance distributions corresponding to each pair of features (fa, fb). (step 16) and
- listing all the molecules (mol2) of the potential library sorted by similarity score value for which their similarity score with respect to the reference compound (mol1) is less than a specified threshold (step 17).

9. A method according to claim 4, **characterized in that** specific chemical groups involved are checked, considering that:

- aliphatic amino groups are under cationic form, while aromatic amines are taken as neutral;
- a special flag is used to signal the presence of imidazole rings, which may appear under physiological conditions, at neutral pH, under both protonated or unprotonated form;
- carboxylate, sulphonate, phosphate groups and tetrazoles are considered to be anions.

10. A method according to claim 5, **characterized in that** steric hindrance criteria are then applied in order to check whether the spacekeeper strategy has managed to insure an appropriate accessibility for the reactive center for every conformation, these criteria comprising :

- a comparison of the interatomic distances in the different conformations in order to make sure that the retained conformers are not redundant;
- an energy criterion discarding higher-energy conformers found to have an almost identical geometry with respect to lower-energy conformations.

11. A method according to claim 8, **characterized in that** the classification of the calculated interatomic distances for every pair of pharmacophoric features is done in a "fuzzy" manner, in order to avoid classification artifacts.

12. A method according to anyone of the preceding claims, **characterized in that** the reactivity profile includes transformers to be coupled to the reactive center of a first building block prior to its coupling to a second building block, each synthesis protocol involving functional transformer to be appended to the first building block.

13. A method according to anyone of the preceding claims, **characterized in that** the bulky spacekeeper group is a tris(triiodosilyl)methyl-entity.

14. A method according to anyone of the preceding claims, **characterized in that**, the number of pharmacophoric features being six, a generated conformational fingerprint vector describing the current geometry is represented with a 252-element vector.

15. A method according to anyone of the preceding claims, **characterized by** a superposition test consisting in trying to superpose the conformers of each selected molecule on the conformers of lead compound, in counting the number of functional groups of the lead compound which are covered by similar groups of the selected molecule and in selecting the molecules having good fingerprint similarity scores and good superposition scores.

16. A method according to anyone of the preceding claims, **characterized in that** synthesis of the retrieved structures from the potential library are performed, and the structures are subjected to biological testing, and that a list of all the building blocks represented in the retrieved products is established and a generation focused combinatorial library is based on such building blocks.

17. A library of potential combinatorial products, **characterized in that** each potential combinatorial product is represented in this library by its molecular fingerprint vector obtained as disclosed in anyone of the preceding claims and by identification codes of the radicals that compose this product.

18. A method of identifying analogs of a compound, comprising the preparation of the fingerprint of said compound, and the screening of the library of claim 17, for similarity scoring.

19. A method of identifying analogs of a compound, comprising the preparation of the fingerprint of said compound, and the screening of the library of claim 17, by recursive partitioning.

20. The use of Fingerprints as defined in any one of claims 1 to 16, as molecular descriptors for data-mining approaches, such as recursive partitioning or other quantitative structure-activity relationships.

**Patentansprüche**

1. Verfahren zum schnellen Auffinden von ähnlichen und daher potentiell aktiven synthetischen Analoga einer Führungsverbindung aus einer Referenzbibliothek von Bausteinverbindungen und aus Syntheseprotokollen für die kombinatorische Synthese dieser Analoga, wobei das Verfahren die folgenden Schritte umfasst:

- Bausteine in der Referenzbibliothek mittels eines chemischen Filteralgorithmus umfassend elementare chemische Regeln und gegebenenfalls Modelle zur Vorhersage von Reaktivitäten basierend auf spezifischen Reaktivitätsdeskriptoren auszuwählen, wobei die ausgewählten Bausteine gültige Reaktionspartner in den chemischen Verfahren der kombinatorischen Synthese sind, ihre reaktiven Zentren nachzuweisen und ihre molekularen Skizzen in Reste umzuwandeln, wobei die Reste die Substrukturen sind, die die kombinatorischen Produkte ausmachen;

- 3D-multikonformative Modelle dieser Reste zu bauen, indem sie temporär in einer 2D-Skizze mit einer sperrigen Platzhalter-Gruppe verbunden werden und der entstehende Komplex einem konformativen Probenlauf unterworfen wird, so dass eine Sammlung von 3D-Konformeren erhalten werden, anschließend den Platzhalter zu entfernen, um sicherzustellen, dass die reaktiven Zentren der Konformeren sterisch zugänglich sind und ihre freien Valenzen auf eine Region eines freien Platzes gerichtet sind, der zuvor durch den Platzhalter belegt war;

- diese 3D-Konformeren in einer 3D-Reste-Datenbank zu registrieren, nachdem überprüft worden ist, dass sie bestimmte sterische Hinderungs- und konformative Diversitätskriterien erfüllen, wobei die Datenbank als Input für eine "Geisterdatenbank" kombinatorischer Produkte dient, wobei die "Geisterdatenbank" einen Algorithmus umfasst, der eine Datenbank mit kombinatorischen Produkten dadurch erzeugt, dass die registrierten Konformeren der eingesetzten Reste miteinander verbunden werden;

- für jede molekulare Struktur, die innerhalb der "Geisterdatenbank" zugänglich ist, jedes Atom nachzuweisen, das Merkmale einer physikalischen Eigenschaft des pharmakophoren Typs auf Basis der elementaren Regeln zeigt, die die chemische Natur dieser Atome und die molekulare Umgebung, in der sie vorliegen, ausmachen, wobei diese pharmakophoren Merkmale an der Bestimmung der Intensität der zwischenmolekularen Wechselwirkungen beteiligt sind und mindestens einige der Eigenschaften hydrophob, aromatisch, Wasserstoffbrücken-Donor, Wasserstoffbrücken-Akzeptor, anionisch und kationisch umfassen, und alle möglichen Paare pharmakophorer Merkmale, in denen jedes Atom eines dieser pharmakophoren Merkmale zeigt, aufzulisten;

- für jedes Paar von pharmakophoren Merkmalen, die in der molekularen Struktur nachgewiesen worden sind, alle Abstände zwischen den betroffenen Atomen in jeder Konformation dieses Moleküls zu berechnen, den berechneten Abständen auf unscharfe (fuzzy) Weise eine Vielzahl von Abstandsklassen zuzuweisen und eine Abstandsverteilungsdichte zu erstellen;

- einen konformativen Fingerabdruck-Vektor zu erzeugen, der die Abstandsverteilungsdichen aller Paare von pharmakophoren Merkmalen, die mit einer gegenwärtigen Konformation des Moleküls assoziiert sind, enthalten, aus den konformativen Fingerabdrücken aller berücksichtigten Konformationen einen durchschnittlichen molekularen Fingerabdruck zu berechnen und diesen durchschnittlichen molekularen Fingerabdruck zu registrieren und dadurch eine potenzielle Bibliothek zu erstellen und bereitzustellen;

- eine Bewertungsfunktion (scoring function) zu definieren, um ein Maß der Ähnlichkeit zwischen zwei molekularen Fingerabdrücken zu ermitteln, die relative Wichtigkeit der pharmakophoren Merkmale in Folge von wichtenden Faktoren, die kalibriert sind, um die Unterscheidungskraft in Bezug auf verschiedene Bindungsaffinitäten zu maximieren, zu begründen; und

- die Fingerabdrücke der Führungsverbindung zu erzeugen, diese Fingerabdrücke mit jedem molekularen Fingerabdruck der potentiellen Bibliothek nach der oben beschriebenen Bewertungsfunktion zu vergleichen und die Moleküle der potentiellen Bibliothek, für die die Bewertungsfunktion Werte unterhalb einer bestimmten Schwelle ergibt, in der Ordnung ansteigender Werte aufzulisten.

2. Das Verfahren nach Anspruch 1, **dadurch gekennzeichnet, dass** die wichtenden Faktoren der Bewertungsfunktion kalibriert werden, um die Unterscheidungskraft zwischen Familien von Liganden verschiedener Rezeptoren in einem "allgemeinen Diversitätsparadigma" zu maximieren, indem als objektive Funktion die Anzahl der Rezeptoren, für die mindestens ein Ligand in der diversesten Auswahl herausgesucht worden ist, verwendet wird.

3. Das Verfahren nach Anspruch 1, **dadurch gekennzeichnet, dass** die wichtenden Faktoren der Aufnahmefunktion kalibriert werden, um die Unterscheidungskraft zwischen den Verbindungen, die an einen bestimmten Rezeptor binden, im Vergleich zu den Verbindungen, die keine Bindungsaffinität zu ihm haben, in einem "Rezeptor-orientierten Diversitätsparadigma" mittels primären Durchsuchens der Ergebnisse einer Bibliothek gegen einen bestimmten Rezeptor zu maximieren, um damit den durchschnittlichen Abstand zwischen zwei aktiven Verbindungen zu minimieren und die Unterschiedlichkeitsbewertungen zwischen einer aktiven und einer inaktiven Verbindung zu maximieren.

4. Das Verfahren nach einem der vorhergehenden Ansprüche, **dadurch gekennzeichnet, dass** es vorläufige Untersuchungsschritte umfasst, um gegenwärtige Bausteine, die potentiell interferierende aktive Gruppen enthalten könnten, zu verwerfen und/oder funktionelle Gruppen vor der Kupplung einem Baukasten zuzufügen.

5. Das Verfahren nach einem der vorhergehenden Ansprüche, **dadurch gekennzeichnet, dass** der Schritt, 3D-Konformere zu erzeugen, ergänzt wird durch einen Schritt der kodierenden strukturellen Information über einen geometrischen Filter durch eine Extraktion aus einer konformativen schnellen Probenanalyse von molekularen Strukturen von 2D-Skizzen der ausgewählten Reste in Verbindung mit pharmakophoren Merkmalen und in Bezug auf jede spezielle chemische Gruppe, die bei jedem Rest involviert ist, um verbleibende Konformationen wichtiger sterischer Hinderungen um das reaktive Zentrum herum und mit anderen Proben ähnlichen Konformationen nachzuweisen und zu verwerfen.

**6.** Das Verfahren nach einem der vorhergehenden Ansprüche, **dadurch gekennzeichnet, dass** die in einer Referenzbibliothek gegenwärtig verfügbaren Bausteine und der Satz an Syntheseprotokollen regelmäßig auf den neuesten Stand gebracht werden, wobei jedes dieser Auf-den-neuesten-Stand-Bringen automatisch das Auf-den-neuesten-Stand-Bringen der potentiellen Bibliotheken auslöst.

**7.** Das Verfahren nach Anspruch 1, **dadurch gekennzeichnet, dass** es integriert ist in ein Nachweisparadigma, das definiert ist als eine Rückführschleife ausgehend von einem primären, "blinden" Durchsuchen einer Bibliothek von Verbindungen, und wobei das Verfahren die Schritte umfasst, aktive Verbindungen zu identifizieren, Parameter eines vorhersagenden Modells auszubilden und anzupassen, um dadurch die spezifischen Merkmale von aktiven Bindungen zu erkennen, potentiell aktive Analoga für diese Verbindungen nach dem vorliegenden Verfahren aus einer Referenzbibliothek aufzufinden, diese Analoga zu synthetisieren und ein Screening durchzuführen, um die Parameter zu verfeinern.

**8.** Das Verfahren nach einem der vorhergehenden Ansprüche, **dadurch gekennzeichnet, dass** es im Wesentlichen die folgenden Schritte umfasst:

- ein Reaktivitätsprofil der laufenden Synthese, bestehend aus erforderlichen und interferierenden Gruppen, aufzuzeichnen (Schritt 1);
- zu prüfen, ob der ausgewählte Baustein ein einzelnes Molekül enthält, und die Gegenionen zu entfernen (Schritt 2);
- die Gegenwart von interferierenden Gruppen in dem ausgewählten Baustein zu überprüfen und diesen gegenwärtigen Baustein zu verwerfen (Schritt 3);
- die Gegenwart von erforderlichen funktionellen Gruppen in dem Baustein zu überprüfen und das reaktive Zentrum zu ermitteln, das an der Bindung mit einem zweiten Baustein unter Bildung von reaktiven Partner-Resten (S1/S2) beteiligt ist, und die Abgangsgruppen zu eliminieren (Schritt 4);
- dem Schweratom-Gerüst der Skizze eines jeden Rests (S1/S2) Wasserstoff zuzufügen, wobei wenigstens sechs pharmakophore Merkmale, nämlich die Aromatizität, die Hydrophobizität, die Wasserstoffbrücken-Donor- oder -Akzeptor-Eigenschaft sowie die positive und negative Ladung eines jeden Atoms überprüft und aufgelistet werden (Schritt 6);
- die 2D-Skizze des Rests mit seiner freien Valenz an der sperrigen Platzhalter-Gruppe zu verankern (Schritt 7);
- die resultierende 2D-Skizze dieser Verbindung einem bekannten konformativen Probenlauf zu unterwerfen und eine Sammlung von Konformeren zu erzeugen (Schritt 8);
- für jedes der erhaltenen Konformeren (Schritt 8) die Platzhalter-Einheit zu trennen und zu zerstören, die freie Valenz des Rests, die nun auf die freie Region, die zuvor durch den Platzhalter besetzt war, gerichtet ist, wieder herzustellen (Schritt 9);
- für jedes zurückgehaltene Konformere eine Kooardinaten-Transformation in einem Referenzsystem (OXYZ) durchzuführen, um das reaktive Zentrum in den Ursprung des Referenzsystems zu legen und die freie Valenz entlang der Z-Achse auszurichten (Schritt 9);
- eine Liste all derjenigen Verbindungen zu erzeugen, die durch Kupplung von jedem Rest (S1) mit jedem seiner Partner (S2) erhalten werden (Schritt 10);
- über alle aufgelisteten Paare (Schritt 10) einen Bogen zu schlagen und die gegenwärtigen Paare von Resten (S1/S2) zusammen mit all ihren verfügbaren Konformeren wie erhalten (Schritt 9) und die Listen der pharmakophoren Merkmale der zuvor verwendeten zusammensetzenden Atome zu verwahren, wobei die pharmakophoren Merkmale der mittels neuer Verbindungen miteinander verbundenen Atome neu ermittelt werden (Schritt 11);
- jedes Konformere (S1i) des ersten Rests (S1) mit jedem Konformeren (S2j) des zweiten Rests (S2) miteinander zu verbinden, indem die Koordinaten des zweiten Rests bezüglich der XOY-Ebene gespiegelt werden, die Koordinaten entlang einer Achse (Z) zu übersetzen, um die richtige Länge der neu gebildeten Bindung zwischen den Konformeren wieder herzustellen und um die neue Achse zu rotieren (Schritt 12);
- einen kompletten Satz interatomarer Abstände für die gegenwärtige Konformation eines jeden Dimers zu ermitteln, um die Abstandsverteilungsdichte von Paaren von pharmakophoren Merkmalen zu konstruieren, wobei alle Abstände zwischen Atompaaren, die mit einem vorgegebenen Paar von pharmakophoren Merkmalen übereinstimmen, in Abstandsklassen klassifiziert werden (Schritt 13);
- einen erzeugten konformativen Fingerabdruck-Vektor, der die gegenwärtige Geometrie mit einer Reihe von Elementen bezüglich der Zahl der Kombinationen beschreibt, die mit den pharmakophoren Merkmalen erhalten werden können, darzustellen; wobei jedes Fingerabdruck-Element (FP) mit der Anzahl an Atompaaren, die mit einem vorgegebenen Paar von pharmakophoren Merkmalen (fa, fb) übereinstimmen und die durch einen Abstand getrennt werden, der innerhalb eine oben genannte Abstandsklasse fällt, gleich ist (Schritt 14);

- die konformativen Fingerabdrücke zu summieren und dadurch einen molekularen Fingerabdruck-Vektor zu erhalten, diese Summe bezüglich der Zahl der berücksichtigten Konformeren des Produktes zu normieren, um somit einen durchschnittlichen molekularen Fingerabdruck zu erhalten, der in Verbindung mit den Identifikationscodes der Reste, die das Produkt zusammensetzen, verwahrt wird; wobei die Sammlung aller Fingerabdrücke aller möglichen Kopplungsprodukte zwischen allen Bausteinen die anfänglichen Syntheseverfahren bezeichnet und potentielle Bibliotheken definiert (Schritt 14);
- Führungs- oder bekannte Liganden-Strukturen unter der Form von Fingerabdrücken zu kodieren, wobei das oben beschriebene Verfahren (Schritt 14) durchgeführt wird, und wobei die Konformeren verwendet werden, die durch den bekannten konformativen Probenlauf (Schritt 8) erhalten werden, dem die Skizzen dieser Referenz-Verbindungen als solche unterworfen werden (Schritt 15);
- eine Bewertungsfunktion einzuführen, die sukzessive die Abstandsverteilungen entsprechend jedem Paar von Merkmalen (fa, fb) vergleicht (Schritt 16) und
- alle Moleküle (mol2) der potentiellen Bibliothek, sortiert nach ihrer Ähnlichkeitsbewertung, deren Ähnlichkeit bezüglich der Referenz-Verbindung (mol1) geringer ist als eine spezifizierte Schwelle, aufzulisten (Schritt 17).

9. Das Verfahren nach Anspruch 4, **dadurch gekennzeichnet, dass** spezifische chemische Gruppen, die involviert sind, unter Berücksichtigung folgender Aspekte überprüft werden:

   - aliphatische Aminogruppen werden als kationisch, aromatische Amine hingegen als neutral angesehen;
   - eine spezielle Markierung wird verwendet, um die Gegenwart von Imidazolringen zu kennzeichnen, die unter physiologischen Bedingungen und bei neutralem pH entweder protoniert oder unprotoniert vorliegen können;
   - Carboxylat, Sulfonat-, Phosphat-Gruppen und Tetrazole werden als Anionen betrachtet.

10. Das Verfahren nach Anspruch 5, **dadurch gekennzeichnet, dass** Kriterien der sterischen Hinderung angewendet werden, um zu überprüfen, ob die Platzhalter-Strategie erfolgreich war, um für jede Konformation eine geeignete Zugänglichkeit für das reaktive Zentrum sicherzustellen, wobei diese Kriterien umfassen:

    - einen Vergleich der interatomaren Abstände in den verschiedenen Konformationen, um sicherzustellen, dass die verbleibenden Konformeren nicht redundant sind;
    - ein Energiekriterium, das Konformere höherer Energie verwirft, von denen gefunden wurde, dass sie eine nahezu identische Geometrie bezüglich der Konformationen mit geringerer Energie besitzen.

11. Das Verfahren nach Anspruch 8, **dadurch gekennzeichnet, dass** die Klassifikation der kalkulierten interatomaren Abstände für jedes Paar von pharmakophoren Merkmalen auf unscharfe Weise passiert, um Klassifikationsartefakte zu verhindern.

12. Das Verfahren nach einem der vorhergehenden Ansprüche, **dadurch gekennzeichnet, dass** das Reaktivitätsprofil Umwandler (Transformer) umfasst, die an das reaktive Zentrum eines ersten Bausteins gekoppelt werden sollen, ehe sie an einen zweiten Baustein gekoppelt werden, wobei jedes Syntheseprotokoll an den ersten Baustein anzuhängende funktionale Umwandler (Transformer) beinhaltet.

13. Das Verfahren nach einem der vorhergehenden Ansprüche, **dadurch gekennzeichnet, dass** die sperrige Platzhalter-Gruppe eine Tris-(trijodsilyl)-methyl-Einheit ist.

14. Das Verfahren nach einem der vorhergehenden Ansprüche, **dadurch gekennzeichnet, dass** ein erzeugter konformativer Fingerabdruck-Vektor, der die gegenwärtige Geometrie beschreibt, mit einem 252-Elemente-Vektor dargestellt wird, wobei die Zahl der pharmakophoren Merkmale sechs ist.

15. Das Verfahren nach einem der vorhergehenden Ansprüche, **gekennzeichnet durch** einen Superpositionstest, der daraus besteht, zu versuchen, die Konformeren eines jeden ausgewählten Moleküls auf die Konformeren der Führungsverbindung zu legen, die Zahl der funktionellen Gruppen der Führungsverbindung, die **durch** ähnliche Gruppen des ausgewählten Moleküls abgedeckt werden, zu bestimmen und die Moleküle mit guten Fingerabdruck-Ähnlichkeitswerten und guten Superpositionswerten auszuwählen.

16. Das Verfahren nach einem der vorhergehenden Ansprüche, **dadurch gekennzeichnet, dass** die Synthese der in der potentiellen Bibliothek aufgefundenen Strukturen erfolgt und einem biologischen Test unterworfen werden, und dass eine Liste aller Bausteine, die in den aufgefundenen Produkten repräsentiert sind, erstellt wird und eine auf die Erzeugung fokusierte kombinatorische Bibliothek auf solchen Bausteinen beruht.

**17.** Bibliothek potentieller kombinatorischer Produkte, **dadurch gekennzeichnet, dass** jedes potentielle kombinatorische Produkt in dieser Bibliothek dargestellt ist durch seinen molekularen Fingerabdruck-Vektor, erhalten wie in einem der vorhergehenden Ansprüche offenbart, und mittels Identifizierungscodes der Reste, die dieses Produkt zusammensetzen.

**18.** Verfahren zur Identifizierung von Analoga einer Verbindung, wobei das Verfahren die Herstellung eines Fingerabdrucks der Verbindung und das Durchsuchen der Bibliothek nach Anspruch 17 mit dem Zweck, Ähnlichkeiten zu finden, umfasst.

**19.** Verfahren zur Identifizierung von Analoga einer Verbindung, wobei das Verfahren die Herstellung eines Fingerabdrucks der Verbindung und das Durchsuchen der Bibliothek nach Anspruch 17 durch rekursives Unterteilen umfasst.

**20.** Verwendung von Fingerabdrücken wie in einem der Ansprüche 1 bis 16 definiert, als molekulare Deskriptoren Data-Mining-Ansätze wie rekursives Unterteilen oder andere quantitative Struktur-Aktivitätsbeziehungen.

**Revendications**

**1.** Procédé pour récupérer rapidement des analogues synthétiques similaires, et donc potentiellement actifs, d'un composé tête de série à partir d'une librairie de référence de composés synthons et de protocoles de synthèse pour la synthèse combinatoire desdits analogues, ledit procédé comprenant les étapes de :

- sélection de synthons dans la librairie de référence par un algorithme à filtre chimique comprenant les règles chimiques élémentaires et, éventuellement, les modèles de prédictions de réactivités basés sur des descripteurs de réactivités spécifiques, les synthons sélectionnés étant des partenaires de réaction valides dans les procédés chimiques de synthèse combinatoire, détection de leurs centres réactifs et conversion de leurs modèles moléculaires en radicaux, lesdits radicaux étant des sous-structures qui conduisent aux produits combinatoires ;
- construction des modèles 3D-multiconformationels de ces radicaux en les liant temporairement dans une présentation-2D à un groupe volumineux maintenant un espace et en soumettant les complexes résultants à un échantillonnage conformationnel donnant lieu à une collection de conformères 3D, puis retrait du groupe maintenant un espace afin de permettre aux centres réactifs des conformères d'être stériquement accessibles et à leurs points de valences d'être libres au regard d'une région d'espace libre précédemment occupée par le groupe maintenant un espace ;
- enregistrement de ces conformères 3D, après vérification qu'ils correspondent à certains critères d'encombrements stériques et critères de diversité conformationnelle, dans une base de données de radicaux 3D qui sert d'entrée pour une « base de données virtuelle » de produits combinatoires, la « base de données virtuelle » comprenant un algorithme générant une base de données de produits combinatoires en liant ensemble les conformères enregistrés des radicaux les constituant ;
- pour toutes les structures moléculaires qui sont accessibles dans la base de données virtuelle, détection d'un atome qui présente des caractéristiques de propriété physique du type pharmacophorique sur la base des règles élémentaires justifiant la nature chimique d'un tel atome et l'environnement moléculaire dans lequel il est placé, ces caractéristiques pharmacophoriques étant impliquées dans la détermination de l'intensité d'interactions intermoléculaires et comprenant au moins quelques caractères hydrophobique, aromatique, de donneur de liaison hydrogène, d'accepteur de liaison hydrogène, anionique et cationique, et listage de toutes les paires possibles de caractéristiques pharmacophoriques dans lesquelles chaque atome présente une desdites caractéristiques pharmacophoriques ;
- pour chaque paire de caractéristiques pharmacophoriques détectée dans ladite structure moléculaire, calcul de toutes les distances entre les atomes impliqués dans chaque conformation de cette molécule, attribution de manière « floue » desdites distances calculées à une pluralité de classes de distance et création d'une densité de distributions de distances ;
- génération d'un vecteur d'empreinte conformationnelle qui contient les densités de distributions de distances de toutes les paires de caractéristiques pharmacophoriques associées à une conformation habituelle de la molécule, calcul d'une empreinte moléculaire moyenne à partir des empreintes conformationnelles de toutes les conformations considérées et enregistrement de cette empreinte moléculaire moyenne pour constituer et fournir une librairie potentielle ;
- définition d'une fonction de notation pour évaluer une mesure de similarité entre deux empreintes moléculaires, justifiant l'importance relative de caractéristiques pharmacophoriques dues à des facteurs de pondération qui sont calibrés afin de maximiser un pouvoir discriminant au regard des différentes affinités de liaisons ; et

- génération d'empreintes du composé tête de série, comparaison de ces empreintes à chaque empreinte moléculaire de la librairie potentielle selon la fonction de notation indiquée ci-dessus et listage afin d'augmenter les valeurs de notation des molécules de la librairie potentielle pour lesquelles la fonction de notation donne des valeurs d'évaluation plus basses qu'un seuil spécifié.

2. Méthode selon la revendication 1, **caractérisée en ce que** les facteurs de pondération de la fonction de notation sont calibrés afin de maximiser le pouvoir discriminant entre les familles de ligands de différents récepteurs dans un « paradigme de diversité générale » en utilisant comme fonction objective le nombre de récepteurs pour lequel au moins un ligand a été identifié dans la sélection la plus diverse.

3. Méthode selon la revendication 1, **caractérisée en ce que** les facteurs de pondération de la fonction de notation sont calibrés pour optimiser le pouvoir discriminant des composés qui se lient à un récepteur donné par rapport à ceux qui n'ont pas d'affinité de liaison pour ce récepteur, dans un « paradigme de diversité orienté-récepteur » en criblant en premier lieu des résultats d'une librairie testée sur un récepteur donné, ce qui permet de minimiser la distance moyenne entre deux composés actifs et d'optimiser les notations de différences entre chaque composé actif et n'importe quel composé inactif.

4. Méthode selon l'une quelconque des revendications précédentes, **caractérisée en ce qu'**elle inclut des étapes préliminaires de contrôle afin de supprimer les synthons usuels qui pourraient potentiellement inclure des groupes réactifs interférants et/ou d'ajouter des groupes fonctionnels à un synthon avant le couplage.

5. Méthode selon l'une quelconque des revendications précédentes, **caractérisée en ce que** la construction de conformères 3D est réalisée avec une étape d'information structurale codante sur un filtre géométrique en extrayant à partir d'une analyse d'échantillonage rapide conformationnel des structures moléculaires de présentations-2D des radicaux sélectionnés en rapport avec les caractéristiques pharmacophoriques et au regard de chaque groupe chimique spécifique impliqué dans chaque radical pour détecter et supprimer les conformations restantes d'encombrement stérique important autour du centre réactif et des conformations similaires à d'autres composés échantillonnés.

6. Méthode selon l'une quelconque des revendications précédentes, **caractérisée en ce que** les synthons disponibles habituellement dans une librairie de référence et un jeu de protocoles de synthèse sont régulièrement mis à jour, chacune de ces mises à jour déclenchant automatiquement la mise à jour des librairies potentielles.

7. Méthode selon la revendication 1, **caractérisée en ce qu'**elle est intégrée dans un paradigme de découverte définissant une boucle de réaction partant d'un criblage « aveugle », et primaire d'une librairie de composés, et incluant les étapes d'identification de composés actifs, d'entraînement et d'ajustement des paramètres d'un modèle prédictif pour reconnaître les caractéristiques spécifiques de composés actifs, de retrait d'analogues potentiellement actifs pour ces composés à partir d'une librairie de référence selon la présente invention, de synthèse de ces analogues et de criblage pour affiner les paramètres.

8. Méthode selon l'une quelconque des revendications précédentes, **caractérisée en ce qu'**elle comprend essentiellement les étapes suivantes :

- scanner un profil réactif de synthèse habituelle consistant en des groupes requis et des groupes interférants (étape 1) ;
- vérifier si le synthon sélectionné contient une molécule unique et supprimer les contre-ions (étape 2) ;
- vérifier la présence de groupe interférant dans le synthon sélectionné et supprimer de tel synthon usuel (étape 3) ;
- vérifier la présence de groupes fonctionnels requis dans le synthon et trouver le centre réactif impliqué dans la liaison avec un second synthon pour former des radicaux partenaires réactifs (S1/S2), éliminer les groupes restants (étape 4) ;
- ajouter des hygrogènes au squelette d'atome lourd des présentations de chaque radical (S1/S2), au moins six caractéristiques pharmacophoriques, propriété d'aromaticité, hydrophobicité, donneur ou accepteur de liaison hydrogène, charge positive ou négative de chaque atome, étant vérifiées et listées (étape 6) ;
- accrocher la présentation-2D du radical avec sa valence libre au groupe volumineux maintenant un espace (étape 7) ;

- soumettre la présentation-2D résultant de ce composé à un échantillonage conformationnel connu, donnant lieu à une collection de conformères (étape 8) ;
- séparer et supprimer, pour chaque conformère obtenu (étape 8), la portion du groupe maintenant un espace, restaurer la valence libre du radical qui se dirige maintenant vers la région vide précédemment occupée par le groupe maintenant un espace (étape 9) ;
- réaliser, pour chaque conformère retenu, une transformation en coordonnées dans un système de référence (OXYZ), afin de placer le centre réactif dans l'origine du système de référence et d'aligner la valence libre le long de l'axe Z (étape 9) ;
- générer une liste de tous les composés qui sont obtenus par couplage de chaque radical (S1) avec chacun de ses partenaires (S2) (étape 10) ;
- boucler toutes les paires listées (étape 10) et stocker les paires habituelles de radicaux (S1, S2) ensemble avec tous les conformères disponibles, ainsi obtenus (étape 9) et les listes des caractéristiques pharmacophoriques des atomes les composant précédemment utilisés, les caractéristiques pharmacophoriques des atomes liés par de nouvelles liaisons étant évaluées de nouveau (étape 11) ;
- lier chaque conformère (S1i) du premier radical (S1) avec chaque conformère (S2j) du second radical (S2) par réflexion des coordonnées de ce dernier par rapport au plan XOY, par traduction de celles-ci le long de l'axe (Z) pour restaurer la longueur exacte de la liaison nouvellement formée entre les conformères ; et par rotation autour du nouvel axe (étape 12) ;
- pour construire la densité de distribution de distances de paires de caractéristiques pharmacophoriques, évaluer un jeu complet de distances interatomiques pour la conformation courante de chaque dimère ; toutes les distances entre paires d'atomes qui correspondent à une paire donnée de caractéristiques pharmacophoriques étant classifiées en classes de distance (étape 13) ;
- représenter un vecteur d'empreinte conformationnelle généré décrivant la géométrie courante avec un nombre d'éléments au regard du nombre de combinaisons qui peuvent être obtenues avec les caractéristiques pharmacophoriques, chaque élément d'empreinte (EE) étant égal au nombre de paires d'atomes correspondant à une paire donnée de caractéristiques pharmacophoriques (ca, cb) et qui sont séparées par une distance qui tombe dans des classes de distance mentionnées ci-dessus (étape 14) ;
- faire la somme des empreintes conformationnelles pour obtenir un vecteur d'empreinte moléculaire et normer cette somme au regard des nombres de conformères considérés du produit pour obtenir une empreinte moléculaire moyenne qui est sauvegardée en association avec des codes d'identification des radicaux qui composent ce produit ; la récupération de toutes les empreintes de tous les produits de couplage possibles entre tous les synthons qualifiant les procédés de synthèse initiaux et définissant les librairies potentielles (étape 14) ;
- coder les têtes de série ou les structures de ligand connues, sous la forme d'empreintes, en suivant la procédure ci-dessus (étape 14), mais en utilisant les conformères obtenus par ledit échantillonnage conformationnel connu, auquel les représentations de ces composés de référence sont soumises tel que (étape 15) ;
- introduire une fonction de notation, qui successivement compare les distributions de distances correspondant à chaque paire de caractéristiques (ca, cb) (étape 16) et
- établir une liste de toutes les molécules (mol2) de la librairie potentielle triée par valeur de notation similaire, pour laquelle la notation de similitude au regard du composé de référence (mol1) est moins élevée qu'un seuil spécifié (étape 17).

9. Méthode selon la revendication 4, **caractérisée en ce que** les groupes chimiques spécifiques impliqués sont vérifiés, en considérant que :

- les groupes amino aliphatiques sont sous une forme cationique, alors que les amines aromatiques sont considérés comme neutres ;
- un marquage spécial est utilisé pour signaler la présence de cycles imidazole, qui peuvent apparaître à des conditions physiologiques, à pH neutre, sous la forme à la fois protonée ou non protonée ;
- les groupes carboxylate, sulfonate, phosphate et tetrazole sont considérés comme des anions.

10. Méthode selon la revendication 5, **caractérisée en ce que** les critères d'encombrement stérique sont ensuite appliqués afin de vérifier si la stratégie du groupement maintenant un espace a permis d'assurer une accessibilité appropriée pour le centre réactif pour chaque conformation, ces critères comprenant :

- une comparaison des distances interatomiques dans les différentes conformations pour s'assurer que les conformères retenus ne sont pas redondants ;
- un critère d'énergie écartant les conformères de haute énergie trouvés comme ayant une géométrie presque identique aux conformères de basse énergie.

**11.** Méthode selon la revendication 8, **caractérisée en ce que** la classification des distances interatomiques calculées pour chaque paire de caractéristiques pharmacophoriques est faite de manière « floue » pour éviter des artéfacts de classification.

**12.** Méthode selon l'une quelconque des revendications précédentes, **caractérisée en ce que** le profil de réactivité inclut des transformateurs à coupler au centre réactif d'un premier synthon avant son couplage à un second synthon, chaque protocole de synthèse impliquant un transformateur fonctionnel à joindre au premier synthon.

**13.** Méthode selon l'une quelconque des revendications précédentes, **caractérisée en ce que** le groupe volumineux maintenant un espace est une entité tris(triiodosilyl)méthyl.

**14.** Méthode selon l'une quelconque des revendications précédentes, **caractérisée en ce que**, le nombre de caractéristiques pharmacophoriques étant de six, un vecteur d'empreinte conformationnelle généré décrivant la géométrie courante est représenté avec un vecteur à 252 éléments.

**15.** Méthode selon l'une quelconque des revendications précédentes, **caractérisée en ce que** le test de superposition consistant à essayer de superposer les conformères de chaque molécule sélectionnée sur les conformères du composé tête de série, en comptant le nombre de groupes fonctionnels du composé tête de série qui sont couverts par des groupes similaires du composé sélectionné et en sélectionnant les molécules ayant de bonnes notations de similitude d'empreinte et de superposition.

**16.** Méthode selon l'une quelconque des revendications précédentes, **caractérisée en ce que** les synthèses des structures extraites à partir d'une librairie potentielle sont réalisées, et lesdites structures sont sujettes à des tests biologiques, et **en ce qu'**une liste de tous les synthons représentés dans les produits extraits est établie et une production d'une librairie combinatoire est faite sur la base de tels synthons.

**17.** Librairie de produits combinatoires potentiels, **caractérisée en ce que** chaque produit combinatoire potentiel est représenté dans cette librairie par son vecteur d'empreinte moléculaire tel que décrit dans l'une quelconque des revendications précédentes et par codes d'identification des radicaux qui composent ce produit.

**18.** Méthode d'identification d'analogues d'un composé, comprenant la préparation de l'empreinte dudit composé et le criblage de la librairie de la revendication 17, pour noter la similitude.

**19.** Méthode d'identification d'analogues d'un composé, comprenant la préparation de l'empreinte dudit composé et le criblage de la librairie de la revendication 17, par partitionnement récurrent.

**20.** Utilisation d'empreintes tels que définies selon l'une quelconque des revendications 1 à 16, comme descripteurs pour des approches d'exploration de données, telles que le partitionnement récurrent ou d'autres relations quantitatives structure-activité.

| STEP 1 | REACTIVITY PROFILE | |
|---|---|---|
| STEP 2 | COUNTERIONS DELETION | |
| STEP 3 | INTERFERING GROUPS | I CHEMICAL FILTER |
| STEP 4 | REACTIVE CENTER DETECTION | |
| STEP 5 | TRANSFORMATION | |

| STEP 6 | CHECK OF PHARMACOPHORIC FEATURES | |
|---|---|---|
| STEP 7 | SPACEKEEPER ANCHORING MODELING | |
| STEP 8 | CONFORMERS COLLECTION | II GEOMETRICAL FILTER |
| STEP 9 | SPACEKEEPER SEVERING AND CONFORMER POSITIONNING | |
| STEP 10 | LIST OF PAIRS OF RADICALS | |

FIGURE 1

STEP 10     LIST OF PAIRS OF RADICALS

STEP 11     GHOST DATABASE LOOPING OVER PAIRS OF RADICALS

STEP 12     CONFORMER BONDING AND ROTATING

III COMBINATORIAL GHOST DATABASE

STEP 13     DISTANCE DISTRIBUTION DENSITY

STEP 14     CONFORMATIONAL FINGERPRINTS AND MOLECULAR FINGERPRINT

FINGERPRINT CONSTRUCTION

STEP 15     LEAD FINGERPRINT VECTORS

STEP 16     SCORING FUNCTION SIMILARITY SCORE

IV COMPARISON ALGORITHM

STEP 17     SPECIFIED THRESHOLD SORTING FUNCTION

FIGURE 2

FIGURE 3

FIGURE 4

(A)

(B)

(C)

(D)

FIGURE 5

S1

FIGURE 6

(II)

R=

(E)

(F)

(G)

(H)

(I)

(J)

(K)

(e)

FIGURE 7

FIGURE 8